# EUROPEAN PATENT APPLICATION

(11) **EP 4 725 421 A2**
(43) Date of publication of application: **15.04.2026**
(21) Application number: 26154600.6
(22) Date of filing: 22.09.2022
(51) Int. Cl.: A61B 5/377

(54) **SYSTEM FOR EVOKING AND RECORDING NEURAL RESPONSES**

(30) Priority: 13.10.2021 US 202163255165 P
(62) Divisional of application: 22790130.3
(71) Applicant: Boston Scientific Neuromodulation Corporation, Valencia, CA 91355 (US)
(72) Inventor: STEINKE, G. Karl, Valencia, California, 91355 (US); SERRANO CARMONA, Paul Enrique, Venice, California, 90291 (US); HADDOCK, Andrew James, Los Angeles, California, 90027 (US); MALEKMOHAMMADI, Mahsa, Sherman Oaks, California, 91411 (US); ESTELLER, Rosana, Santa Clarita, California, 91390 (US)
(74) Representative: Vossius & Partner Patentanwälte Rechtsanwälte mbB

(57) **Abstract**

The present invention relates to a system for delivering neurostimulation to a patient using a plurality of electrodes. The system comprises a stimulation output circuit configured to deliver the neurostimulation to evoke responses from the patient using an evoking configuration defining a stimulation electrode set including electrodes selected from the plurality of electrodes; and a sensing input circuit configured to sense one or more signals including the evoked responses using a recording configuration defining a sensing electrode set including electrodes selected from the plurality of electrodes. The system further comprises a control circuit configured to control the delivery of the neurostimulation using stimulation parameters defining the evoking configuration, to control the sensing of the evoked responses using sensing parameters defining the recording configuration, and to adjust the stimulation parameters and the sensing parameters according to a sequence of evoking-recording parameter sets each including a set of the stimulation parameters determined for controlling the stimulation output circuit to deliver the neurostimulation to evoke a target response and a set of the sensing parameters determined for controlling the sensing input circuit to record the evoked target response.

## Description

### CLAIM OF PRIORITY

This application claims the benefit of U.S. Provisional Application No. 63/255,165, filed on October 13, 2021, which is hereby incorporated by reference in its entirety.

### TECHNICAL FIELD

This document relates generally to neurostimulation and more particularly to a neurostimulation system that provides for efficient determination of stimulation and sensing parameters controlling evoking and sensing of neural responses.

### BACKGROUND

Neurostimulation, also referred to as neuromodulation, has been proposed as a therapy for a number of conditions. Examples of neurostimulation include Spinal Cord Stimulation (SCS), Deep Brain Stimulation (DBS), Peripheral Nerve Stimulation (PNS), and Functional Electrical Stimulation (FES). Implantable neurostimulation systems have been applied to deliver such a therapy. An implantable neurostimulation system may include an implantable neurostimulator, also referred to as an implantable pulse generator (IPG), and one or more implantable leads each including one or more electrodes. The implantable neurostimulator delivers neurostimulation energy through one or more electrodes placed on or near a target site in the nervous system. An external programming device is used to program the implantable neurostimulator with stimulation parameters controlling the delivery of the neurostimulation energy.

In one example, the neurostimulation energy is delivered in a form of electrical pulses. The delivery is controlled using stimulation parameters that specify spatial (where to stimulate), temporal (when to stimulate), and informational (patterns of pulses directing the nervous system to respond as desired) aspects of the electrical pulses. Signals indicative of the patient's response to the delivery of the electrical pulses may be sensed for setting and adjusting the stimulation parameters. For example, a neural signal indicative of the patient's response may be sensed for verifying lead placement, optimizing the stimulation parameters, and/or closed-loop control of a neurostimulation therapy may be sensed to allow for closed-loop control of its delivery. Efficacy and safety of a neurostimulation therapy may depend on customization of initial settings for the patient and continuing adjustments of the settings for the patient's changing conditions, which in turn depend on proper sensing of the patient's responses to the therapy.

### SUMMARY

An example (e.g., "Example 1") of a system for delivering neurostimulation to a patient using a plurality of electrodes is provided. The system may include a stimulation output circuit, a sensing input circuit, and a control circuit. The stimulation output circuit may be configured to deliver the neurostimulation to evoke responses from the patient using an evoking configuration defining a stimulation electrode set including electrodes selected from the plurality of electrodes. The sensing input circuit may be configured to sense one or more signals including the evoked responses using a recording configuration defining a sensing electrode set including electrodes selected from the plurality of electrodes. The control circuit may include a stimulation controller, a sensing controller, and a parameter adjuster. The stimulation controller may be configured to control the delivery of the neurostimulation using stimulation parameters including the evoking configuration. The sensing controller may be configured to control the sensing of the evoked responses using sensing parameters including the recording configuration. The parameter adjuster may be configured to determine an evoking-recording parameter set by evaluating a sequence of test evoking-recording parameter sets. The evoking-recording parameter set may include a set of the stimulation parameters suitable for controlling the stimulation output circuit to deliver the neurostimulation to evoke a target response and a set of the sensing parameters suitable for controlling the sensing input circuit to record the evoked target response.

In Example 2, the subject matter of Example 1 may optionally be configured such that the parameter adjuster is configured to determine the evoking-recording parameter set for controlling the stimulation output circuit and the sensing input circuit in deep brain stimulation.

In Example 3, the subject matter of any one or any combination of Examples 1 and 2 may optionally be configured such that the stimulation output circuit includes multiple stimulation channels, and the evoking configuration defines a different stimulation electrode set for each channel of the multiple stimulation channels to allow for simultaneous delivery of the neurostimulation using different stimulation electrode sets.

In Example 4, the subject matter of any one or any combination of Examples 1 to 3 may optionally be configured such that the sensing input circuit includes multiple sensing channels, and the recording configuration defines a different sensing electrode set for each channel of the multiple sensing channels to allow for simultaneous sensing of multiple signals using different sensing electrode sets.

In Example 5, the subject matter of Example 4 may optionally be configured such that the parameter adjuster is configured to evaluate multiple test evoking-recording parameter sets of the sequence of test evoking-recording parameter sets simultaneously using two or more sensing channels of the multiple sensing channels.

In Example 6, the subject matter of any one or any combination of Examples 1 to 5 may optionally be configured such that the evoking configuration further defines a fractionalization specifying a distribution of a current of the neurostimulation over the stimulation electrode set.

In Example 7, the subject matter of any one or any combination of Examples 1 to 6 may optionally be configured such that the stimulation electrode set includes electrodes selected from the plurality of electrodes for monopolar stimulation.

In Example 8, the subject matter of any one or any combination of Examples 1 to 7 may optionally be configured such that the stimulation electrode set includes electrodes selected from the plurality of electrodes for bipolar stimulation.

In Example 9, the subject matter of any one or any combination of Examples 1 to 8 may optionally be configured such that the stimulation electrode set includes electrodes selected from the plurality of electrodes for anodic stimulation.

In Example 10, the subject matter of any one or any combination of Examples 1 to 9 may optionally be configured such that the stimulation output circuit is configured to deliver pulses of the neurostimulation, and the stimulation controller is configured to control the delivery of the pulses using the stimulation parameters including the evoking configuration and waveform parameters defining waveform of the pulses.

In Example 11, the subject matter of any one or any combination of Examples 1 to 10 may optionally be configured such that the parameter adjuster is configured to identify an optimal evoking-recording parameter set from the sequence of test evoking-recording parameter sets.

In Example 12, the subject matter of any one or any combination of Examples 1 to 11 may optionally be configured such that the parameter adjuster is configured to reduce a number of the test evoking-recording parameter sets in the sequence of test evoking-recording parameter sets using prior information.

In Example 13, the subject matter of Example 12 may optionally be configured such that the parameter adjuster is configured to reduce a number of changes in the recording configuration in the sequence of test evoking-recording parameter sets.

In Example 14, the subject matter of any one or any combination of Examples 1 to 13 may optionally be configured such that the parameter adjuster is configured to evaluate each test evoking-recording parameter set of the sequence of test evoking-recording parameter sets by causing the stimulation output circuit to deliver a burst of neurostimulation pulses and the sensing input circuit to sense the one or more signals using the each test evoking-recording parameter set and to minimize a number of pulses in the burst of neurostimulation pulses while allowing for reliable sensing of the evoked target response.

In Example 15, the subject matter of any one or any combination of Examples 1 to 14 may optionally be configured such that each test evoking-recording parameter set in the sequence of test evoking-recording parameter sets includes a pair of the stimulation electrode set and the sensing electrode set, the sensing electrode set of the pair defined by an electrode type and a location relative to the stimulation electrode set of the pair.

An example (e.g., "Example 16") of a method for delivering neurostimulation to a patient using a plurality of electrodes is also provided. The method may include delivering the neurostimulation to evoke responses from the patient using a stimulation output circuit and an evoking configuration defining a stimulation electrode set including electrodes selected from the plurality of electrodes, sensing one or more signals including the evoked responses using a sensing input circuit and a recording configuration defining a sensing electrode set including electrodes selected from the plurality of electrodes, controlling the delivery of the neurostimulation using stimulation parameters including the evoking configuration, controlling the sensing of the one or more signals including the evoked responses using sensing parameters including the recording configuration, and determining an evoking-recording parameter set by evaluating a sequence of test evoking-recording parameter sets. The evoking-recording parameter set may include a set of the stimulation parameters suitable for controlling the delivery of the neurostimulation to evoke a target response and a set of the sensing parameters suitable for controlling the sensing of the one or more signals to record the evoked target response.

In Example 17, the subject matter of determining the evoking-recording parameter set as found in Example 16 may optionally include determining the evoking-recording parameter set for deep brain stimulation.

In Example 18, the subject matter of determining the evoking-recording parameter set as found in Example 17 may optionally include determining the evoking-recording parameter set for recording evoked resonant neural activity (ERNA).

In Example 19, the subject matter of sensing the one or more signals as found in any one or any combination of Examples 16 to 18 may optionally include sensing multiple signals simultaneously using multiple sensing channels of the sensing input circuit and multiple sensing configurations each defining a different sensing electrode set including electrodes selected from the plurality of electrodes, and the subject matter of evaluating the sequence of test evoking-recording parameter sets as found in any one or any combination of Examples 16 to 18 may optionally include evaluating multiple test evoking-recording parameter sets simultaneously using the multiple sensing channels of the sensing input circuit.

In Example 20, the subject matter of delivering the neurostimulation as found in Example 19 may optionally include delivering the neurostimulation simultaneously using multiple stimulation channels of the stimulation output circuit and multiple evoking configurations each defining a different stimulation electrode set including electrodes selected from the plurality of electrodes, and the subject matter of evaluating the sequence of test evoking-recording parameter sets as found in Example 19 may optionally include evaluating multiple test evoking-recording parameter sets simultaneously using the multiple channels of the sensing input circuit and the multiple stimulation channels of the stimulation output circuit.

In Example 21, the subject matter of the evoking configuration as found in any one or any combination of Examples 16 to 20 may optionally include the stimulation electrode set including electrodes selected from the plurality of electrodes and a fractionalization specifying a distribution of a current of the neurostimulation over the stimulation electrode set.

In Example 22, the subject matter of any one or any combination of Examples 16 to 21 may optionally further include selecting the stimulation electrode set from a reference electrode of the plurality of electrodes and lead electrodes of the plurality of electrodes. The lead electrodes each include one or more contacts and incorporated onto a lead configured to be coupled to the stimulation output circuit. The subject matter of controlling the delivery of the neurostimulation as found in any one or any combination of Examples 16 to 21 may optionally include controlling an electrical current of the neurostimulation individually for each of the reference electrode and the contacts of the lead electrodes.

In Example 23, the subject matter of selecting the stimulation electrode set as found in Example 22 may optionally include one or more lead electrodes of the lead electrodes to function as a cathode for delivering the neurostimulation.

In Example 24, the subject matter of selecting the stimulation electrode set as found in Example 23 may optionally further include selecting the reference electrode to function as an anode for delivering the neurostimulation.

In Example 25, the subject matter of selecting the stimulation electrode set as found in Example 23 may optionally further include selecting one or more additional lead electrodes of the lead electrodes to function as an anode for delivering the neurostimulation.

In Example 26, the subject matter of selecting the stimulation electrode set as found in Example 23 may optionally further include selecting the reference electrode and one or more additional lead electrodes of the lead electrodes to function as an anode for delivering the neurostimulation.

In Example 27, the subject matter of determining the evoking-recording parameter set as found in any one or any combination of Examples 16 to 26 may optionally include optimizing the evoking-recording parameter set for evoking and recording a specified target response.

In Example 28, the subject matter of determining the evoking-recording parameter set as found in any one or any combination of Examples 16 to 26 may optionally include determining an evoking-recording configuration including a pair of the evoking configuration and the recording configuration and a stimulation waveform parameter set, and the subject matter of evaluating the sequence of test evoking-recording parameter sets as found in any one or any combination of Examples 16 to 26 may optionally include evaluating a sequence of test evoking-recording configurations and a sequence of test stimulation waveform parameter sets for each test evoking-recording configuration of the sequence of test evoking-recording configurations.

In Example 29, the subject matter of determining the evoking-recording parameter set as found in Example 28 may optionally include: evaluating the sequence of test evoking-recording parameter sets by delivering a burst of pulses of the neurostimulation and sensing the one or more signals according to each test evoking-recording parameter set of the sequence of test evoking-recording parameter sets; and selecting the evoking-recording parameter set from the sequence of test evoking-recording parameter sets based on a result of the evaluation.

In Example 30, the subject matter of the plurality of electrodes as found in Example 29 may optionally include lead electrodes incorporated onto one or more leads configured to be coupled to the stimulation output circuit, and the subject matter of evaluating the sequence of test evoking-recording parameter sets as found in Example 29 may optionally include evaluating test evoking-recording parameter sets including multiple evoking-recording configurations using electrodes selected from lead electrodes incorporated onto a lead of the one or more leads.

In Example 31, the subject matter of any one or a combination of Examples 29 and 30 may optionally further include minimizing a number of pulses in each burst of the bursts of pulses.

In Example 32, the subject matter of any one or a combination of Examples 29 to 31 may optionally further include determining a waveform for the pulses of the neurostimulation to minimize stimulus artifacts.

In Example 33, the subject matter of evaluating the sequence of test evoking-recording parameter sets as found in any one or any combination of Examples 29 to 32 may optionally include evaluating the sequence of test evoking-recording parameter sets according to a temporal order of the test evoking-recording parameter sets specified by the sequence of test evoking-recording parameter sets, and the subject matter of any one or any combination of Examples 29 to 32 may optionally further include determining the temporal order for reducing a duration of the evaluation of the sequence of test evoking-recording parameter sets.

In Example 34, the subject matter of determining the temporal order as found in Example 33 may optionally include minimizing a number of the evoking-recording configurations in the sequence of test evoking-recording parameter sets.

In Example 35, the subject matter of determining the temporal order as found in Example 33 may optionally include determining the evoking-recording configurations in the sequence of test evoking-recording parameter sets using prior information.

In Example 36, the subject matter of determining the temporal order as found in any one or any combination of Examples 33 to 35 may optionally include evaluating two or more evoking-recording configurations in the sequence of test evoking-recording parameter sets simultaneously using at least one of multiple sensing channels of the sensing input circuit and multiple stimulation channels of the stimulation output circuit.

In Example 37, the subject matter of determining the temporal order as found in any one or any combination of Examples 33 to 36 may optionally include minimizing a number of changes in the recording configuration in the sequence of test evoking-recording parameter sets.

In Example 38, the subject matter of determining the temporal order as found in Example 37 may optionally include determining the temporal order for evaluating all test stimulation parameter sets for each evoking-recording configuration before switching to next evoking-recording configuration in the sequence of test evoking-recording parameter sets.

In Example 39, the subject matter of determining the temporal order as found in Example 37 may optionally include determining the temporal order for evaluating all the test evoking-recording parameter sets having a common recording configuration before evaluating test evoking-recording parameter sets having a different recording configuration.

An example (e.g., "Example 40") of a non-transitory computer-readable storage medium including instructions, which when executed by a system, cause the system to perform a method for delivering neurostimulation to a patient using a plurality of electrodes is also provided. The method may include delivering the neurostimulation to evoke responses from the patient using a stimulation output circuit and an evoking configuration defining a stimulation electrode set including electrodes selected from the plurality of electrodes, sensing one or more signals including the evoked responses using a sensing input circuit and a recording configuration defining a sensing electrode set including electrodes selected from the plurality of electrodes, controlling the delivery of the neurostimulation using stimulation parameters including the evoking configuration, controlling the sensing of the one or more signals including the evoked responses using sensing parameters including the recording configuration, and determining an evoking-recording parameter set by evaluating a sequence of test evoking-recording parameter sets. The evoking-recording parameter set may include a set of the stimulation parameters suitable for controlling the delivery of the neurostimulation to evoke a target response and a set of the sensing parameters suitable for controlling the sensing of the one or more signals to record the evoked target response.

Another example (e.g., "Example 41") of a system for delivering neurostimulation to a patient using a plurality of electrodes is provided. The system may include a stimulation output circuit, a sensing input circuit, and a control circuit. The stimulation output circuit may be configured to deliver the neurostimulation to evoke responses from the patient using an evoking configuration defining a stimulation electrode set including electrodes selected from the plurality of electrodes. The sensing input circuit may be configured to sense one or more signals including the evoked responses using a recording configuration defining a sensing electrode set including electrodes selected from the plurality of electrodes. The control circuit may be configured to control the delivery of the neurostimulation using stimulation parameters defining the evoking configuration, to control the sensing of the evoked responses using sensing parameters defining the recording configuration, and to adjust the stimulation parameters and the sensing parameters according to a sequence of evoking-recording parameter sets each including a set of the stimulation parameters determined for controlling the stimulation output circuit to deliver the neurostimulation to evoke a target response and a set of the sensing parameters determined for controlling the sensing input circuit to record the evoked target response.

In Example 42, the subject matter of Example 41 may optionally be configured such that the control circuit is configured to determine an evoking-recording parameter set of the sequence of evoking-recording parameter sets for controlling the stimulation output circuit and the sensing input circuit in deep brain stimulation.

In Example 43, the subject matter of any one or any combination of Examples 41 and 42 may optionally be configured such that the stimulation output circuit includes multiple stimulation channels, and the evoking configuration defines a different stimulation electrode set for each channel of the multiple stimulation channels to allow for simultaneous delivery of the neurostimulation using different stimulation electrode sets.

In Example 44, the subject matter of any one or any combination of Examples 41 to 43 may optionally be configured such that the sensing input circuit includes multiple sensing channels, and the recording configuration defines a different sensing electrode set for each channel of the multiple sensing channels to allow for simultaneous sensing of multiple signals using different sensing electrode sets.

In Example 45, the subject matter of Example 44 may optionally be configured such that the control circuit is configured to evaluate multiple evoking-recording parameter sets of the sequence of evoking-recording parameter sets simultaneously using two or more sensing channels of the multiple sensing channels.

In Example 46, the subject matter of any one or any combination of Examples 41 to 45 may optionally be configured such that the evoking configuration further defines a fractionalization specifying a distribution of a current of the neurostimulation over the stimulation electrode set.

In Example 47, the subject matter of any one or any combination of Examples 41 to 46 may optionally be configured such that the stimulation electrode set includes electrodes selected from the plurality of electrodes for monopolar stimulation.

In Example 48, the subject matter of any one or any combination of Examples 41 to 47 may optionally be configured such that the stimulation electrode set includes electrodes selected from the plurality of electrodes for bipolar stimulation.

In Example 49, the subject matter of any one or any combination of Examples 41 to 48 may optionally be configured such that the stimulation electrode set includes electrodes selected from the plurality of electrodes for anodic stimulation.

In Example 50, the subject matter of any one or any combination of Examples 41 to 49 may optionally be configured such that the stimulation output circuit is configured to deliver pulses of the neurostimulation, and the control circuit is configured to control the delivery of the pulses using the stimulation parameters including the evoking configuration and waveform parameters defining waveform of the pulses.

In Example 51, the subject matter of any one or any combination of Examples 41 to 50 may optionally be configured such that the control circuit is configured to identify an optimal evoking-recording parameter set from the sequence of evoking-recording parameter sets.

In Example 52, the subject matter of any one or any combination of Examples 41 to 51 may optionally be configured such that the control circuit is configured to reduce a number of the evoking-recording parameter sets in the sequence of evoking-recording parameter sets using prior information.

In Example 53, the subject matter of Example 52 may optionally be configured such that the control circuit is configured to reduce a number of changes in the recording configuration in the sequence of evoking-recording parameter sets.

In Example 54, the subject matter of any one or any combination of Examples 41 to 53 may optionally be configured such that the control circuit is configured to evaluate each evoking-recording parameter set of the sequence of evoking-recording parameter sets by causing the stimulation output circuit to deliver a burst of neurostimulation pulses and the sensing input circuit to sense the one or more signals using the each evoking-recording parameter set and to minimize a number of pulses in the burst of neurostimulation pulses while allowing for reliable sensing of the evoked target response.

In Example 55, the subject matter of any one or any combination of Examples 41 to 54 may optionally be configured such that each evoking-recording parameter set in the sequence of evoking-recording parameter sets includes a pair of the stimulation electrode set and the sensing electrode set, the sensing electrode set of the pair defined by an electrode type and a location relative to the stimulation electrode set of the pair.

Another example (e.g., "Example 56") of a method for delivering neurostimulation to a patient using a plurality of electrodes is also provided. The method may include delivering the neurostimulation to evoke responses from the patient using a stimulation output circuit and an evoking configuration defining a stimulation electrode set including electrodes selected from the plurality of electrodes, sensing one or more signals including the evoked responses using a sensing input circuit and a recording configuration defining a sensing electrode set including electrodes selected from the plurality of electrodes, controlling the delivery of the neurostimulation using stimulation parameters including the evoking configuration, controlling the sensing of the one or more signals including the evoked responses using sensing parameters including the recording configuration, and adjusting the stimulation parameters and the sensing parameters according to a sequence of evoking-recording parameter sets each including a set of the stimulation parameters determined for controlling the stimulation output circuit to deliver the neurostimulation to evoke a target response and a set of the sensing parameters determined for controlling the sensing input circuit to record the evoked target response.

In Example 57, the subject matter as found in Example 16 may optionally further include determining an evoking-recording parameter set of the sequence of evoking-recording parameter sets for deep brain stimulation.

In Example 58, the subject matter of determining the evoking-recording parameter set as found in Example 57 may optionally include determining the evoking-recording parameter set for recording evoked resonant neural activity (ERNA).

In Example 59, the subject matter of sensing the one or more signals as found in any one or any combination of Examples 56 to 58 may optionally include sensing multiple signals simultaneously using multiple sensing channels of the sensing input circuit and multiple sensing configurations each defining a different sensing electrode set including electrodes selected from the plurality of electrodes, and the subject matter as found in any one or any combination of Examples 56 to 58 may optionally further include evaluating multiple evoking-recording parameter sets of the sequence of evoking-recording parameter sets simultaneously using the multiple sensing channels of the sensing input circuit.

In Example 60, the subject matter of delivering the neurostimulation as found in Example 59 may optionally include delivering the neurostimulation simultaneously using multiple stimulation channels of the stimulation output circuit and multiple evoking configurations each defining a different stimulation electrode set including electrodes selected from the plurality of electrodes, and the subject matter of evaluating the sequence of evoking-recording parameter sets as found in Example 19 may optionally include evaluating multiple evoking-recording parameter sets simultaneously using the multiple channels of the sensing input circuit and the multiple stimulation channels of the stimulation output circuit.

In Example 61, the subject matter of the evoking configuration as found in any one or any combination of Examples 56 to 60 may optionally include the stimulation electrode set including electrodes selected from the plurality of electrodes and a fractionalization specifying a distribution of a current of the neurostimulation over the stimulation electrode set.

In Example 62, the subject matter of any one or any combination of Examples 56 to 61 may optionally further include selecting the stimulation electrode set from a reference electrode of the plurality of electrodes and lead electrodes of the plurality of electrodes. The lead electrodes each include one or more contacts and incorporated onto a lead configured to be coupled to the stimulation output circuit. The subject matter of controlling the delivery of the neurostimulation as found in any one or any combination of Examples 56 to 61 may optionally include controlling an electrical current of the neurostimulation individually for each of the reference electrode and the contacts of the lead electrodes.

In Example 63, the subject matter of selecting the stimulation electrode set as found in Example 62 may optionally include one or more lead electrodes of the lead electrodes to function as a cathode for delivering the neurostimulation.

In Example 64, the subject matter of selecting the stimulation electrode set as found in Example 63 may optionally further include selecting the reference electrode to function as an anode for delivering the neurostimulation.

In Example 65, the subject matter of selecting the stimulation electrode set as found in Example 63 may optionally further include selecting one or more additional lead electrodes of the lead electrodes to function as an anode for delivering the neurostimulation.

In Example 66, the subject matter of selecting the stimulation electrode set as found in Example 63 may optionally further include selecting the reference electrode and one or more additional lead electrodes of the lead electrodes to function as an anode for delivering the neurostimulation.

In Example 67, the subject matter of determining the evoking-recording parameter set as found in any one or any combination of Examples 56 to 66 may optionally include optimizing the evoking-recording parameter set for evoking and recording a specified target response.

In Example 68, the subject matter as found in any one or any combination of Examples 56 to 66 may optionally further include determining an evoking-recording configuration including a pair of the evoking configuration and the recording configuration and a stimulation waveform parameter set, and evaluating a sequence of evoking-recording configurations and a sequence of stimulation waveform parameter sets for each evoking-recording configuration of the sequence of evoking-recording configurations.

In Example 69, the subject matter of determining the evoking-recording parameter set as found in Example 68 may optionally include: evaluating the sequence of evoking-recording parameter sets by delivering a burst of pulses of the neurostimulation and sensing the one or more signals according to each evoking-recording parameter set of the sequence of evoking-recording parameter sets; and selecting the evoking-recording parameter set from the sequence of evoking-recording parameter sets based on a result of the evaluation.

In Example 70, the subject matter of the plurality of electrodes as found in Example 69 may optionally include lead electrodes incorporated onto one or more leads configured to be coupled to the stimulation output circuit, and the subject matter as found in Example 69 may optionally further include evaluating evoking-recording parameter sets including multiple evoking-recording configurations using electrodes selected from lead electrodes incorporated onto a lead of the one or more leads.

In Example 71, the subject matter of any one or a combination of Examples 69 and 70 may optionally further include minimizing a number of pulses in each burst of the bursts of pulses.

In Example 72, the subject matter of any one or a combination of Examples 69 to 71 may optionally further include determining a waveform for the pulses of the neurostimulation to minimize stimulus artifacts.

In Example 73, the subject matter of evaluating the sequence of evoking-recording parameter sets as found in any one or any combination of Examples 69 to 72 may optionally include evaluating the sequence of evoking-recording parameter sets according to a temporal order of the evoking-recording parameter sets specified by the sequence of evoking-recording parameter sets, and the subject matter of any one or any combination of Examples 69 to 72 may optionally further include determining the temporal order for reducing a duration of the evaluation of the sequence of evoking-recording parameter sets.

In Example 74, the subject matter of determining the temporal order as found in Example 73 may optionally include minimizing a number of the evoking-recording configurations in the sequence of evoking-recording parameter sets.

In Example 75, the subject matter of determining the temporal order as found in Example 73 may optionally include determining the evoking-recording configurations in the sequence of evoking-recording parameter sets using prior information.

In Example 76, the subject matter of determining the temporal order as found in any one or any combination of Examples 73 to 75 may optionally include evaluating two or more evoking-recording configurations in the sequence of evoking-recording parameter sets simultaneously using at least one of multiple sensing channels of the sensing input circuit and multiple stimulation channels of the stimulation output circuit.

In Example 77, the subject matter of determining the temporal order as found in any one or any combination of Examples 73 to 76 may optionally include minimizing a number of changes in the recording configuration in the sequence of evoking-recording parameter sets.

In Example 78, the subject matter of determining the temporal order as found in Example 77 may optionally include determining the temporal order for evaluating all stimulation parameter sets for each evoking-recording configuration before switching to next evoking-recording configuration in the sequence of evoking-recording parameter sets.

In Example 79, the subject matter of determining the temporal order as found in Example 77 may optionally include determining the temporal order for evaluating all the evoking-recording parameter sets having a common recording configuration before evaluating evoking-recording parameter sets having a different recording configuration.

Another example (e.g., "Example 80") of a non-transitory computer-readable storage medium including instructions, which when executed by a system, cause the system to perform a method for delivering neurostimulation to a patient using a plurality of electrodes is also provided. The method may include delivering the neurostimulation to evoke responses from the patient using a stimulation output circuit and an evoking configuration defining a stimulation electrode set including electrodes selected from the plurality of electrodes, sensing one or more signals including the evoked responses using a sensing input circuit and a recording configuration defining a sensing electrode set including electrodes selected from the plurality of electrodes, controlling the delivery of the neurostimulation using stimulation parameters including the evoking configuration, controlling the sensing of the one or more signals including the evoked responses using sensing parameters including the recording configuration, and adjusting the stimulation parameters and the sensing parameters according to a sequence of evoking-recording parameter sets each including a set of the stimulation parameters determined for controlling the stimulation output circuit to deliver the neurostimulation to evoke a target response and a set of the sensing parameters determined for controlling the sensing input circuit to record the evoked target response.

This Summary is an overview of some of the teachings of the present application and not intended to be an exclusive or exhaustive treatment of the present subject matter. Further details about the present subject matter are found in the detailed description and appended claims. Other aspects of the disclosure will be apparent to persons skilled in the art upon reading and understanding the following detailed description and viewing the drawings that form a part thereof, each of which are not to be taken in a limiting sense. The scope of the present disclosure is defined by the appended claims and their legal equivalents.

### BRIEF DESCRIPTION OF THE DRANVINGS

The drawings illustrate generally, by way of example, various embodiments discussed in the present document. The drawings are for illustrative purposes only and may not be to scale.
FIG. 1 illustrates an embodiment of a neurostimulation system.
FIG. 2 illustrates an embodiment of a stimulation device and a lead system, such as may be implemented in the neurostimulation system of FIG. 1.
FIG. 3 illustrates an embodiment of a programming device, such as may be implemented in the neurostimulation system of FIG. 1.
FIG. 4 illustrates an embodiment of an implantable pulse generator (IPG) and an implantable lead system, such as an example implementation of the stimulation device and lead system of FIG. 2.
FIG. 5 illustrates an embodiment of an IPG and an implantable lead system, such as the IPG and lead system of FIG. 4, arranged to provide neurostimulation to a patient.
FIG. 6 illustrates an embodiment of portions of a neurostimulation system.
FIG. 7 illustrates an embodiment of an implantable stimulator and one or more leads of an implantable neurostimulation system, such as the implantable neurostimulation system of FIG. 6.
FIG. 8 illustrates an embodiment of an external programming device of an implantable neurostimulation system, such as the implantable neurostimulation system of FIG. 6.
FIG. 9 illustrates an embodiment of a system for evoking and recording neural responses.
FIG. 10A-C illustrate an embodiment of a lead for use with a system for evoking and recording neural responses, such as the system of FIG. 9, with FIG. 10A being a side view of a distal portion of the lead, FIG. 10B being a cross-sectional view showing a 3-segmental electrode, and FIG. 10C being a cross-sectional view showing a ring electrode.
FIG. 11 illustrates an embodiment of electrodes including electrodes on the lead of FIG. 10 and a reference electrode.
FIG. 12A-F illustrate examples of evoking-recording configurations based the electrodes of FIG. 11, with FIGS. 12A and 12B showing first and second examples for monopolar stimulation, respectively, and FIGS. 12C, 12D, 12E, and 12F showing first, second, third, and fourth example for bipolar stimulation, respectively.
FIG. 13A-D illustrate examples of stimulation waveforms for use in a system for evoking and recording neural responses, such as the system of FIG. 9., with FIG. 13A showing an example of a pulse waveform that may cause amplifier saturation, FIGS. 13B, 13C, and 13D showing first, second, and third examples of the pulse waveform modified to reduce or avoid saturation of the recorded signal, respectively.
FIG. 14 illustrates an embodiment of a method for evoking and recording neural responses.

### DETAILED DESCRIPTION

In the following detailed description, reference is made to the accompanying drawings which form a part hereof, and in which is shown by way of illustration specific embodiments in which the invention may be practiced. These embodiments are described in sufficient detail to enable those skilled in the art to practice the invention, and it is to be understood that the embodiments may be combined, or that other embodiments may be utilized, and that structural, logical and electrical changes may be made without departing from the spirit and scope of the present invention. References to "an", "one", or "various" embodiments in this disclosure are not necessarily to the same embodiment, and such references contemplate more than one embodiment. The following detailed description provides examples, and the scope of the present invention is defined by the appended claims and their legal equivalents.

This document discusses, among other things, a method and system for efficiently and effectively determining stimulation and sensing parameters for evoking and recording neural responses using a neurostimulation system. The neurostimulation system can control the sensing of neural signals including evoked responses and use the sensed signals as well as their relationship to stimulation parameters to determine and adjust settings for delivery of neurostimulation and settings for the sensing of neural signals. In various embodiments, the neuromodulation system can include an implantable device configured to deliver neurostimulation (also referred to as neuromodulation) therapies, such as deep brain stimulation (DBS), spinal cord stimulation (SCS), peripheral nerve stimulation (PNS), and vagus nerve stimulation (VNS), and one or more external devices configured to program the implantable device for its operations and monitor the performance of the implantable device. While DBS is specifically discussed as an example, the present subject matter can be applied in various neurostimulation therapies in which neural signals including evoked potentials are sensed.

A neurostimulation system may sense neural signals including spontaneous activity and/or evoked potentials and depend on proper sensing for verifying lead and electrode placement, setting stimulation parameters controlling delivery of neurostimulation pulses for a therapy, and adjusting the stimulation parameters in responses to changing needs and/or conditions of a patient, among other things. Types of neural potentials to be sensed may depend on the type of therapy. For example, in DBS, to avoid undesirable side effects and ensure desirable clinical effects, neural signals may be sensed from the patient's internal brain structure within the limbic system to evaluate local field potentials (LFPs), within the basal ganglia to evaluate evoked potentials (EPs) including evoked resonant neural activity (ERNA), and/or within neocortical areas of the brain to evaluate sensory or motor EPs or motor LFPs signatures. One or more leads each including an array of electrodes may be used for delivering the neurostimulation pulses and sensing the neural signals. The selection of stimulation and sensing configurations, including selecting one or more stimulation electrodes from the array(s) of electrodes for delivering the neurostimulation pulses and selecting one or more sensing electrodes from the array(s) of electrodes for sensing the neural signals, may depend on each type of the neural signals that needs to be evaluated. A neurostimulation system that includes multiple stimulation and sensing channels individually programmable for connecting to different sets of electrodes allow for proper setting or optimization of stimulation and sensing configurations for each type of recorded neural signal, but the process may be time consuming given the numerous possible combinations of stimulation and sensing parameters, including the numerous possible combinations of electrodes selectable to be the stimulation and sensing electrodes. As evoking and recording of evoked neural responses can assist proper placement of electrodes and initial settings of a neurostimulator during an implantation operation, for example, there is a need for an efficient and effective strategy for determining the stimulation and sensing parameters.

The present subject matter provides for determining of the stimulation and sensing parameters suitable for evoking and recording targeted neural responses (including evoked or spontaneous activities) by evaluating various combinations of stimulation and sensing parameters, with various techniques used to reduce the time required for the evaluation. Examples of such techniques include using prior information including patient data and device capabilities to reduce or minimize the number of measurements in the evaluation, using spatial and temporal arrangement of the stimulation and sensing parameters to be evaluated to reduce or minimize total sensing circuit settlement time, etc. In various embodiments, the present subject matter can be applied during placement, adjustment, and operation of an implantable neurostimulation system to establish reliable sensing of targeted neural responses as required by various neurostimulation algorithms such as lead checking, sweet spot (stimulation site) searching, system functionality checking, closed-loop therapy control, and monitoring of effects of medications on evoked potentials.

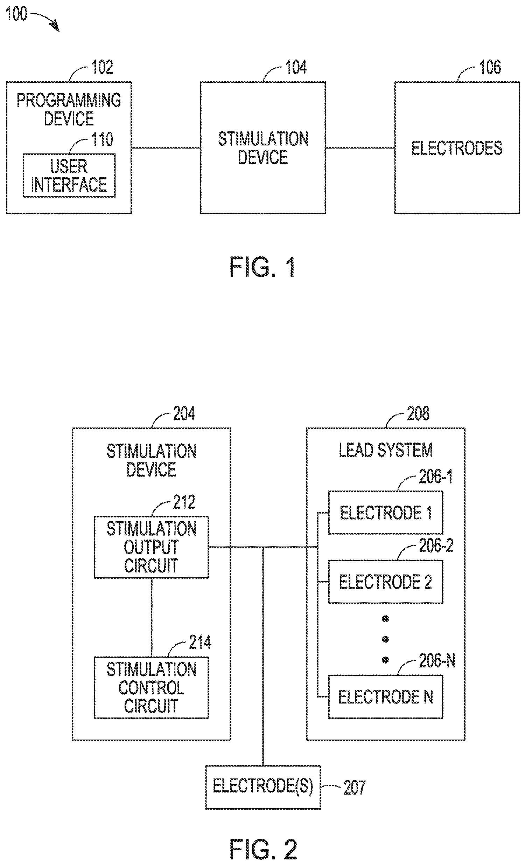

FIG. 1 illustrates an embodiment of a neurostimulation system 100. System 100 includes electrodes 106, a stimulation device 104, and a programming device 102. Electrodes 106 are configured to be placed on or near one or more neural targets in a patient. Stimulation device 104 is configured to be electrically connected to electrodes 106 and deliver neurostimulation energy, such as in the form of electrical pulses, to the one or more neural targets though electrodes 106. The delivery of the neurostimulation is controlled by using a plurality of stimulation parameters, such as stimulation parameters specifying a pattern of the electrical pulses and a selection of electrodes through which each of the electrical pulses is delivered. In various embodiments, at least some parameters of the plurality of stimulation parameters are programmable by a user, such as a physician or other caregiver who treats the patient using system 100. Programming device 102 provides the user with accessibility to the user-programmable parameters. In various embodiments, programming device 102 is configured to be communicatively coupled to stimulation device via a wired or wireless link.

In this document, a "user" includes a physician or other clinician or caregiver who examiners and/or treats the patient using system 100; a "patient" includes a person who receives or is intended to receive neurostimulation delivered using system 100. In various embodiments, the patient can be allowed to adjust his or her treatment using system 100 to certain extent, such as by adjusting certain therapy parameters and entering feedback and clinical effect information.

In various embodiments, programming device 102 can include a user interface 110 that allows the user to control the operation of system 100 and monitor the performance of system 100 as well as conditions of the patient including responses to the delivery of the neurostimulation. The user can control the operation of system 100 by setting and/or adjusting values of the user-programmable parameters.

In various embodiments, user interface 110 can include a graphical user interface (GUI) that allows the user to set and/or adjust the values of the user-programmable parameters by creating and/or editing graphical representations of various waveforms. Such waveforms may include, for example, a waveform representing a pattern of neurostimulation pulses to be delivered to the patient as well as individual waveforms that are used as building blocks of the pattern of neurostimulation pulses, such as the waveform of each pulse in the pattern of neurostimulation pulses. The GUI may also allow the user to set and/or adjust stimulation fields each defined by a set of electrodes through which one or more neurostimulation pulses represented by a waveform are delivered to the patient. The stimulation fields may each be further defined by the distribution of the current of each neurostimulation pulse in the waveform. In various embodiments, neurostimulation pulses for a stimulation period (such as the duration of a therapy session) may be delivered to multiple stimulation fields.

In various embodiments, system 100 can be configured for neurostimulation applications. User interface 110 can be configured to allow the user to control the operation of system 100 for neurostimulation. For example, system 100 as well as user interface 110 can be configured for DBS applications. Such DBS configuration includes various features that may simplify the task of the user in programming stimulation device 104 for delivering DBS to the patient, such as the features discussed in this document.

FIG. 2 illustrates an embodiment of a stimulation device 204 and a lead system 208, such as may be implemented in neurostimulation system 100. Stimulation device 204 represents an embodiment of stimulation device 104 and includes a stimulation output circuit 212 and a stimulation control circuit 214. Stimulation output circuit 212 produces and delivers neurostimulation pulses. Stimulation control circuit 214 controls the delivery of the neurostimulation pulses from stimulation output circuit 212 using the plurality of stimulation parameters, which specifies a pattern of the neurostimulation pulses. Lead system 208 includes one or more leads each configured to be electrically connected to stimulation device 204 and a plurality of electrodes 206 distributed in the one or more leads. The plurality of electrodes 206 includes electrode 206-1, electrode 206-2, ... electrode 206-N, each a single electrically conductive contact providing for an electrical interface between stimulation output circuit 212 and tissue of the patient, where N ≥ 2. The neurostimulation pulses are each delivered from stimulation output circuit 212 through a set of electrodes selected from electrodes 206. In various embodiments, the neurostimulation pulses may include one or more individually defined pulses, and the set of electrodes may be individually definable by the user for each of the individually defined pulses or each of collections of pulse intended to be delivered using the same combination of electrodes. In various embodiments, one or more additional electrodes 207 (each of which may be referred to as a reference electrode) can be electrically connected to stimulation device 204, such as one or more electrodes each being a portion of or otherwise incorporated onto a housing of stimulation device 204. Monopolar stimulation uses a monopolar electrode configuration with one or more electrodes selected from electrodes 206 and at least one electrode from electrode(s) 207. Bipolar stimulation uses a bipolar electrode configuration with two electrodes selected from electrodes 206 and none electrode(s) 207. Multipolar stimulation uses a multipolar electrode configuration with multiple (two or more) electrodes selected from electrodes 206 and none of electrode(s) 207.

In various embodiments, the number of leads and the number of electrodes on each lead depend on, for example, the distribution of target(s) of the neurostimulation and the need for controlling the distribution of electric field at each target. In one embodiment, lead system 208 includes 2 leads each having 8 electrodes.

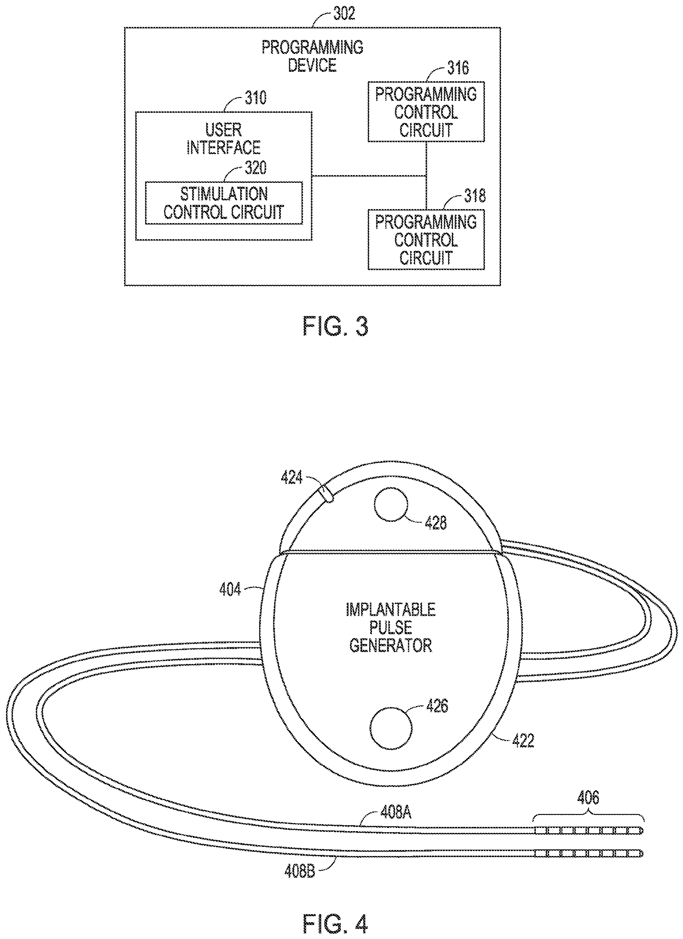

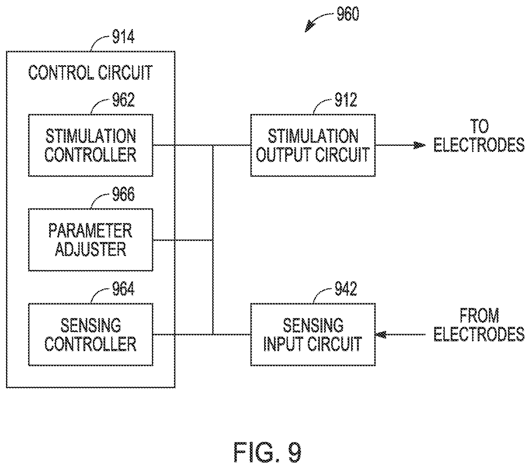

FIG. 3 illustrates an embodiment of a programming device 302, such as may be implemented in neurostimulation system 100. Programming device 302 represents an embodiment of programming device 102 and includes a storage device 318, a programming control circuit 316, and a user interface 310. Programming control circuit 316 generates the plurality of stimulation parameters that controls the delivery of the neurostimulation pulses according to a specified stimulation configuration that can define, for example, stimulation waveform and electrode configuration. User interface 310 represents an embodiment of user interface 110 and includes a stimulation control circuit 320. Storage device 318 stores information used by programming control circuit 316 and stimulation control circuit 320, such as information about a stimulation device that relates the stimulation configuration to the plurality of stimulation parameters and information relating the stimulation configuration to a volume of activation in the patient. In various embodiments, stimulation control circuit 320 can be configured to support one or more functions allowing for programming of stimulation devices, such as stimulation device 104 including its various embodiments as discussed in this document, in evaluating stimulation and sensing parameters for evoking and recording target neural responses, as further discussed below with reference to FIGS. 9-14.

In various embodiments, user interface 310 can allow for definition of a pattern of neurostimulation pulses for delivery during a neurostimulation therapy session by creating and/or adjusting one or more stimulation waveforms using a graphical method. The definition can also include definition of one or more stimulation fields each associated with one or more pulses in the pattern of neurostimulation pulses. As used in this document, a "stimulation configuration" can include the pattern of neurostimulation pulses including the one or more stimulation fields, or at least various aspects or parameters of the pattern of neurostimulation pulses including the one or more stimulation fields. In various embodiments, user interface 310 includes a GUI that allows the user to define the pattern of neurostimulation pulses and perform other functions using graphical methods. In this document, "neurostimulation programming" can include the definition of the one or more stimulation waveforms, including the definition of one or more stimulation fields.

In various embodiments, circuits of neurostimulation 100, including its various embodiments discussed in this document, may be implemented using a combination of hardware and software. For example, the circuit of user interface 110, stimulation control circuit 214, programming control circuit 316, and stimulation control circuit 320, including their various embodiments discussed in this document, may be implemented using an application-specific circuit constructed to perform one or more particular functions or a general-purpose circuit programmed to perform such function(s). Such a general-purpose circuit includes, but is not limited to, a microprocessor or a portion thereof, a microcontroller or portions thereof, and a programmable logic circuit or a portion thereof.

FIG. 4 illustrates an embodiment of an implantable pulse generator (IPG) 404 and an implantable lead system 408. IPG 404 represents an example implementation of stimulation device 204. Lead system 408 represents an example implementation of lead system 208. As illustrated in FIG. 4, IPG 404 that can be coupled to implantable leads 408A and 408B at a proximal end of each lead. The distal end of each lead includes electrical contacts or electrodes 406 for contacting a tissue site targeted for electrical neurostimulation. As illustrated in FIG. 1, leads 408A and 408B each include 8 electrodes 406 at the distal end. The number and arrangement of leads 408A and 408B and electrodes 406 as shown in FIG. 1 are only an example, and other numbers and arrangements are possible. In various embodiments, the electrodes are ring electrodes. The implantable leads and electrodes may be configured by shape and size to provide electrical neurostimulation energy to a neuronal target included in the subject's brain, or configured to provide electrical neurostimulation energy to a nerve cell target included in the subject's spinal cord.

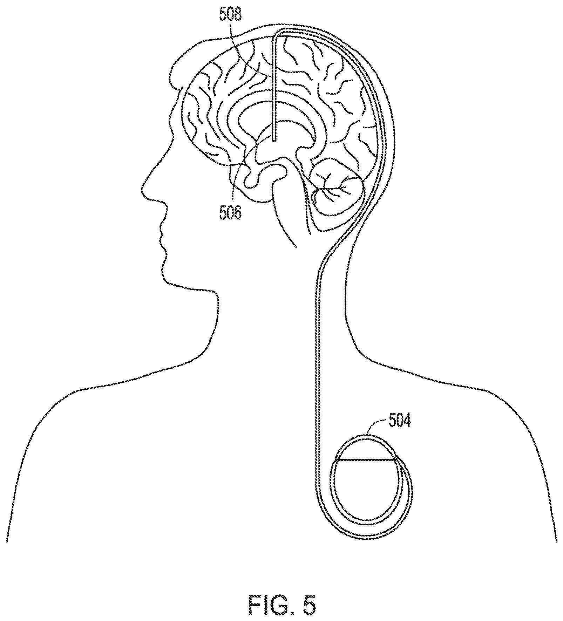

FIG. 5 illustrates an embodiment of an IPG 504 and an implantable lead system 508 arranged to provide neurostimulation to a patient. An example of IPG 504 includes IPG 404. An example of lead system 508 includes one or more of leads 408A and 408B. In the illustrated embodiment, implantable lead system 508 is arranged to provide Deep Brain Stimulation (DBS) to a patient, with the stimulation target being neuronal tissue in a subdivision of the thalamus of the patient's brain. Other examples of DBS targets include neuronal tissue of the globus pallidus (GPi), the subthalamic nucleus (STN), the pedunculopontine nucleus (PPN), substantia nigra pars reticulate (SNr), globus pallidus externus (GPe), medial forebrain bundle (MFB), periaquaductal gray (PAG), periventricular gray (PVG), habenula, subgenual cingulate cortex, ventral intermediate nucleus (VIM) of the thalamus, anterior nucleus (AN) or other nuclei of the thalamus, zona incerta, ventral capsule, ventral striatum, nucleus accumbens, and other white matter tracts connecting these and other structures. DBS is discussed as an example for the present subject matter, which can be applied to other brain stimulation (e.g., cortical stimulation) and other neurostimulation therapies.

Returning to FIG. 4, the IPG 404 can include a hermetically-sealed IPG case 422 to house the electronic circuitry of IPG 404. IPG 404 can include an electrode 426 formed on IPG case 422. In some embodiments, IPG case 422 can be used as electrode 426. IPG 404 can include an IPG header 424 for coupling the proximal ends of leads 408A and 408B. IPG header 424 may optionally also include an electrode 428. Electrodes 426 and/or 428 represent embodiments of electrode(s) 207 and may each be referred to as a reference electrode. Neurostimulation energy can be delivered in a monopolar (also referred to as unipolar) mode using electrode 426 or electrode 428 and one or more electrodes selected from electrodes 406. Neurostimulation energy can be delivered in a bipolar mode using a pair of electrodes of the same lead (lead 408A or lead 408B). Neurostimulation energy can be delivered in an extended bipolar mode using one or more electrodes of a lead (e.g., one or more electrodes of lead 408A) and one or more electrodes of a different lead (e.g., one or more electrodes of lead 408B).

The electronic circuitry of IPG 404 can include a control circuit that controls delivery of the neurostimulation energy. The control circuit can include a microprocessor, a digital signal processor, application specific integrated circuit (ASIC), or other type of processor, interpreting or executing instructions included in software or firmware. The neurostimulation energy can be delivered according to specified (e.g., programmed) modulation parameters. Examples of setting modulation parameters can include, among other things, selecting the electrodes or electrode combinations used in the stimulation, configuring an electrode or electrodes as the anode or the cathode for the stimulation, specifying the percentage of the neurostimulation provided by an electrode or electrode combination, and specifying stimulation pulse parameters. Examples of pulse parameters include, among other things, the amplitude of a pulse (specified in current or voltage), pulse duration (e.g., in microseconds), pulse rate (e.g., in pulses per second), and parameters associated with a pulse train or pattern such as burst rate (e.g., an "on" modulation time followed by an "off" modulation time), amplitudes of pulses in the pulse train, polarity of the pulses, etc.

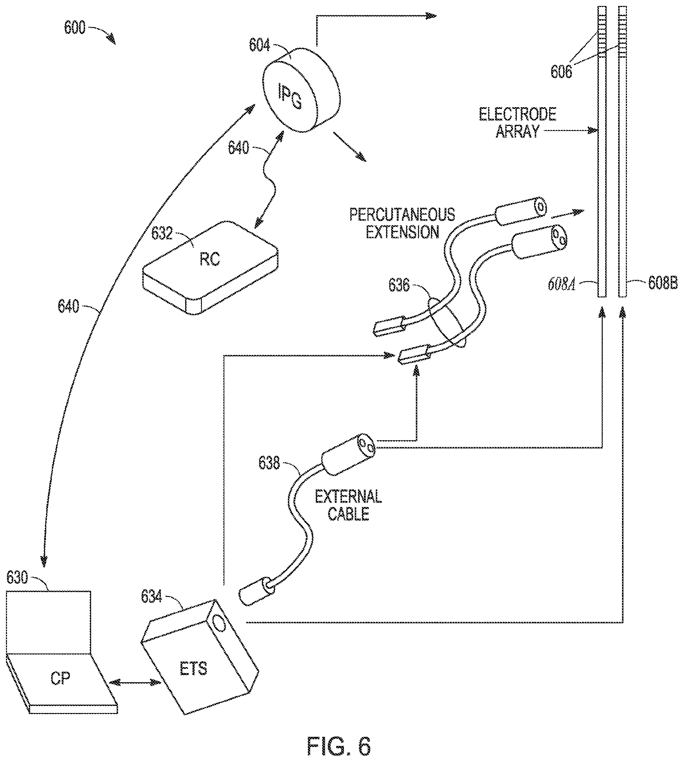

FIG. 6 illustrates an embodiment of portions of a neurostimulation system 600. System 600 includes an IPG 604, implantable neurostimulation leads 608A and 608B, an external remote controller (RC) 632, a clinician's programmer (CP) 630, and an external trial modulator (ETM) 634. IPG 404 may be electrically coupled to leads 608A and 608B directly or through percutaneous extension leads 636. ETM 634 may be electrically connectable to leads 608A and 608B via one or both of percutaneous extension leads 636 and/or external cable 638. System 600 represents an embodiment of system 100, with IPG 604 representing an embodiment of stimulation device 104, electrodes 606 of leads 608A and 608B representing electrodes 106, and CP 630, RC 632, and ETM 634 collectively representing programming device 102.

ETM 634 may be standalone or incorporated into CP 630. ETM 634 may have similar pulse generation circuitry as IPG 604 to deliver neurostimulation energy according to specified modulation parameters as discussed above. ETM 634 is an external device that is typically used as a preliminary stimulator after leads 408A and 408B have been implanted and used prior to stimulation with IPG 604 to test the patient's responsiveness to the stimulation that is to be provided by IPG 604. Because ETM 634 is external it may be more easily configurable than IPG 604.

CP 630 can configure the neurostimulation provided by ETM 634. If ETM 634 is not integrated into CP 630, CP 630 may communicate with ETM 634 using a wired connection (e.g., over a USB link) or by wireless telemetry using a wireless communications link 640. CP 630 also communicates with IPG 604 using a wireless communications link 640.

An example of wireless telemetry is based on inductive coupling between two closely-placed coils using the mutual inductance between these coils. This type of telemetry is referred to as inductive telemetry or near-field telemetry because the coils must typically be closely situated for obtaining inductively coupled communication. IPG 604 can include the first coil and a communication circuit. CP 630 can include or otherwise electrically connected to the second coil such as in the form of a wand that can be place near IPG 604. Another example of wireless telemetry includes a far-field telemetry link, also referred to as a radio frequency (RF) telemetry link. A far-field, also referred to as the Fraunhofer zone, refers to the zone in which a component of an electromagnetic field produced by the transmitting electromagnetic radiation source decays substantially proportionally to 1/r, where r is the distance between an observation point and the radiation source. Accordingly, far-field refers to the zone outside the boundary of r = λ/2π, where λ is the wavelength of the transmitted electromagnetic energy. In one example, a communication range of an RF telemetry link is at least six feet but can be as long as allowed by the particular communication technology. RF antennas can be included, for example, in the header of IPG 604 and in the housing of CP 630, eliminating the need for a wand or other means of inductive coupling. An example is such an RF telemetry link is a Bluetooth^{®} wireless link.

CP 630 can be used to set modulation parameters for the neurostimulation after IPG 604 has been implanted. This allows the neurostimulation to be tuned if the requirements for the neurostimulation change after implantation. CP 630 can also upload information from IPG 604.

RC 632 also communicates with IPG 604 using a wireless link 340. RC 632 may be a communication device used by the user or given to the patient. RC 632 may have reduced programming capability compared to CP 630. This allows the user or patient to alter the neurostimulation therapy but does not allow the patient full control over the therapy. For example, the patient may be able to increase the amplitude of neurostimulation pulses or change the time that a preprogrammed stimulation pulse train is applied. RC 632 may be programmed by CP 630. CP 630 may communicate with the RC 632 using a wired or wireless communications link. In some embodiments, CP 630 is able to program RC 632 when remotely located from RC 632.

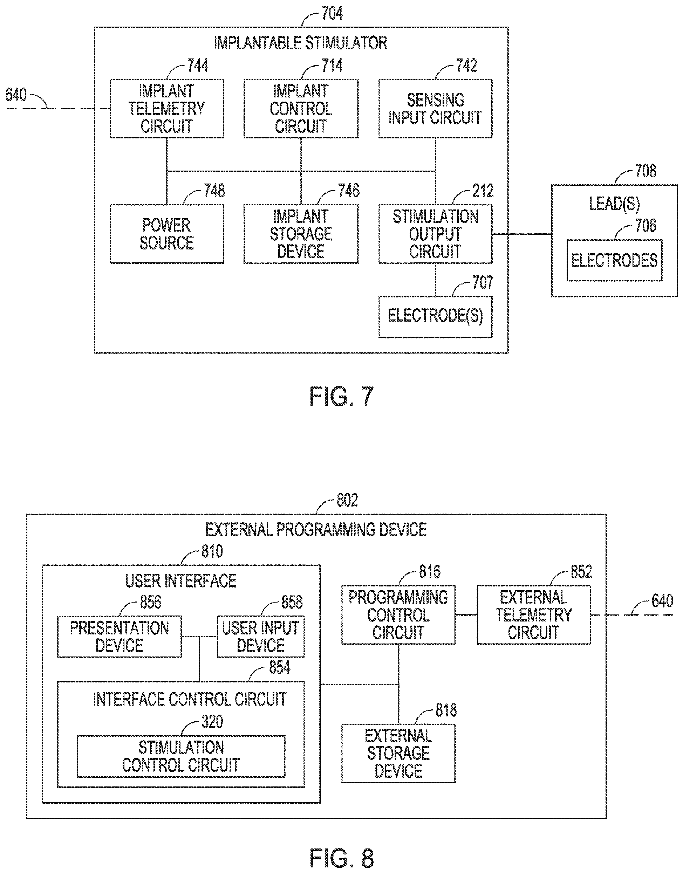

FIG. 7 illustrates an embodiment of implantable stimulator 704 and one or more leads 708 of an implantable neurostimulation system, such as implantable system 600. Implantable stimulator 704 represents an embodiment of stimulation device 104 or 204 and may be implemented, for example, as IPG 604. Lead(s) 708 represents an embodiment of lead system 208 and may be implemented, for example, as implantable leads 608A and 608B. Lead(s) 708 includes electrodes 706, which represents an embodiment of electrodes 106 or 206 and may be implemented as electrodes 606.

Implantable stimulator 704 may include a sensing input circuit (also known as a sensing circuit) 742 that provides the stimulator with a sensing capability, stimulation output circuit 212, a stimulation control circuit 714, an implant storage device 746, an implant telemetry circuit 744, a power source 748, and one or more electrodes 707. Sensing input circuit 742 senses one or more physiological signals for purposes of patient monitoring and/or feedback control of the neurostimulation. Examples of the one or more physiological signals include neural and other signals each indicative of a condition of the patient that is treated by the neurostimulation and/or a response of the patient to the delivery of the neurostimulation. Stimulation output circuit 212 is electrically connected to electrodes 706 through one or more leads 708 as well as electrodes 707, and delivers each of the neurostimulation pulses through a set of electrodes selected from electrodes 706 and electrode(s) 707. Stimulation control circuit 714 represents an embodiment of stimulation control circuit 214 and controls the delivery of the neurostimulation pulses using the plurality of stimulation parameters specifying the pattern of neurostimulation pulses. In one embodiment, stimulation control circuit 714 controls the delivery of the neurostimulation pulses using the one or more sensed physiological signals. Implant telemetry circuit 744 provides implantable stimulator 704 with wireless communication with another device such as CP 630 and RC 632, including receiving values of the plurality of stimulation parameters from the other device. Implant storage device 746 stores values of the plurality of stimulation parameters. Power source 748 provides implantable stimulator 704 with energy for its operation. In one embodiment, power source 748 includes a battery. In one embodiment, power source 748 includes a rechargeable battery and a battery charging circuit for charging the rechargeable battery. Implant telemetry circuit 744 may also function as a power receiver that receives power transmitted from an external device through an inductive couple. Electrode(s) 707 allow for delivery of the neurostimulation pulses in the monopolar mode. Examples of electrode(s) 707 include electrode 426 and electrode 418 in IPG 404 as illustrated in FIG. 4.

In one embodiment, implantable stimulator 704 is used as a master database. A patient implanted with implantable stimulator 704 (such as may be implemented as IPG 604) may therefore carry patient information needed for his or her medical care when such information is otherwise unavailable. Implant storage device 746 is configured to store such patient information. For example, the patient may be given a new RC 632 and/or travel to a new clinic where a new CP 630 is used to communicate with the device implanted in him or her. The new RC 632 and/or CP 630 can communicate with implantable stimulator 704 to retrieve the patient information stored in implant storage device 746 through implant telemetry circuit 744 and wireless communication link 640, and allow for any necessary adjustment of the operation of implantable stimulator 704 based on the retrieved patient information. In various embodiments, the patient information to be stored in implant storage device 746 may include, for example, positions of lead(s) 708 and electrodes 706 relative to the patient's anatomy (transformation for fusing computerized tomogram (CT) of post-operative lead placement to magnetic resonance imaging (MRI) of the brain), clinical effect map data, objective measurements using quantitative assessments of symptoms (for example using micro-electrode recording, accelerometers, and/or other sensors), and/or any other information considered important or useful for providing adequate care for the patient. In various embodiments, the patient information to be stored in implant storage device 746 may include data transmitted to implantable stimulator 704 for storage as part of the patient information and data acquired by implantable stimulator 704, such as by using sensing input circuit 742.

In various embodiments, sensing input circuit 742, stimulation output circuit 212, stimulation control circuit 714, implant telemetry circuit 744, implant storage device 746, and power source 748 are encapsulated in a hermetically sealed implantable housing or case, and electrode(s) 707 are formed or otherwise incorporated onto the case. In various embodiments, lead(s) 708 are implanted such that electrodes 706 are placed on and/or around one or more targets to which the neurostimulation pulses are to be delivered, while implantable stimulator 704 is subcutaneously implanted and connected to lead(s) 708 at the time of implantation.

FIG. 8 illustrates an embodiment of an external programming device 802 of an implantable neurostimulation system, such as system 600. External programming device 802 represents an embodiment of programming device 102 or 302, and may be implemented, for example, as CP 630 and/or RC 632. External programming device 802 includes an external telemetry circuit 852, an external storage device 818, a programming control circuit 816, and a user interface 810.

External telemetry circuit 852 provides external programming device 802 with wireless communication with another device such as implantable stimulator 704 via wireless communication link 640, including transmitting the plurality of stimulation parameters to implantable stimulator 704 and receiving information including the patient data from implantable stimulator 704. In one embodiment, external telemetry circuit 852 also transmits power to implantable stimulator 704 through an inductive couple.

In various embodiments, wireless communication link 640 can include an inductive telemetry link (near-field telemetry link) and/or a far-field telemetry link (RF telemetry link). For example, because DBS is often indicated for movement disorders which are assessed through patient activities, gait, balance, etc., allowing patient mobility during programming and assessment is useful. Therefore, when system 600 is intended for applications including DBS, wireless communication link 640 includes at least a far-field telemetry link that allows for communications between external programming device 802 and implantable stimulator 704 over a relative long distance, such as up to about 20 meters. External telemetry circuit 852 and implant telemetry circuit 744 each include an antenna and RF circuitry configured to support such wireless telemetry.

External storage device 818 stores one or more stimulation waveforms for delivery during a neurostimulation therapy session, such as a DBS therapy session, as well as various parameters and building blocks for defining one or more waveforms. The one or more stimulation waveforms may each be associated with one or more stimulation fields and represent a pattern of neurostimulation pulses to be delivered to the one or more stimulation field during the neurostimulation therapy session. In various embodiments, each of the one or more stimulation waveforms can be selected for modification by the user and/or for use in programming a stimulation device such as implantable stimulator 704 to deliver a therapy. In various embodiments, each waveform in the one or more stimulation waveforms is definable on a pulse-by-pulse basis, and external storage device 818 may include a pulse library that stores one or more individually definable pulse waveforms each defining a pulse type of one or more pulse types. External storage device 818 also stores one or more individually definable stimulation fields. Each waveform in the one or more stimulation waveforms is associated with at least one field of the one or more individually definable stimulation fields. Each field of the one or more individually definable stimulation fields is defined by a set of electrodes through a neurostimulation pulse is delivered. In various embodiments, each field of the one or more individually definable fields is defined by the set of electrodes through which the neurostimulation pulse is delivered and a current distribution of the neurostimulation pulse over the set of electrodes. In one embodiment, the current distribution is defined by assigning a fraction of an overall pulse amplitude to each electrode of the set of electrodes. Such definition of the current distribution may be referred to as "fractionalization" in this document. In another embodiment, the current distribution is defined by assigning an amplitude value to each electrode of the set of electrodes. For example, the set of electrodes may include 2 electrodes used as the anode and an electrode as the cathode for delivering a neurostimulation pulse having a pulse amplitude of 4 mA. The current distribution over the 2 electrodes used as the anode needs to be defined. In one embodiment, a percentage of the pulse amplitude is assigned to each of the 2 electrodes, such as 75% assigned to electrode 1 and 25% to electrode 2. In another embodiment, an amplitude value is assigned to each of the 2 electrodes, such as 3 mA assigned to electrode 1 and 1 mA to electrode 2. Control of the current in terms of percentages allows precise and consistent distribution of the current between electrodes even as the pulse amplitude is adjusted. It is suited for thinking about the problem as steering a stimulation locus, and stimulation changes on multiple contacts simultaneously to move the locus while holding the stimulation amount constant. Control and displaying the total current through each electrode in terms of absolute values (e.g. mA) allows precise dosing of current through each specific electrode. It is suited for changing the current one contact at a time (and allows the user to do so) to shape the stimulation like a piece of clay (pushing/pulling one spot at a time).

Programming control circuit 816 represents an embodiment of programming control circuit 316 and generates the plurality of stimulation parameters, which is to be transmitted to implantable stimulator 704, based on a specified stimulation configuration (e.g., the pattern of neurostimulation pulses as represented by one or more stimulation waveforms and one or more stimulation fields, or at least certain aspects of the pattern). The stimulation configuration may be created and/or adjusted by the user using user interface 810 and stored in external storage device 818. In various embodiments, programming control circuit 816 can check values of the plurality of stimulation parameters against safety rules to limit these values within constraints of the safety rules. In one embodiment, the safety rules are heuristic rules.

User interface 810 represents an embodiment of user interface 310 and allows the user to define the pattern of neurostimulation pulses and perform various other monitoring and programming tasks. User interface 810 includes a display screen 856, a user input device 858, and an interface control circuit 854. Display screen 856 may include any type of interactive or non-interactive screens, and user input device 858 may include any type of user input devices that supports the various functions discussed in this document, such as touchscreen, keyboard, keypad, touchpad, trackball, joystick, and mouse. In one embodiment, user interface 810 includes a GUI. The GUI may also allow the user to perform any functions discussed in this document where graphical presentation and/or editing are suitable as may be appreciated by those skilled in the art.

Interface control circuit 854 controls the operation of user interface 810 including responding to various inputs received by user input device 858 and defining the one or more stimulation waveforms. Interface control circuit 854 includes stimulation control circuit 320.

In various embodiments, external programming device 802 can have operation modes including a composition mode and a real-time programming mode. Under the composition mode (also known as the pulse pattern composition mode), user interface 810 is activated, while programming control circuit 816 is inactivated. Programming control circuit 816 does not dynamically updates values of the plurality of stimulation parameters in response to any change in the one or more stimulation waveforms. Under the real-time programming mode, both user interface 810 and programming control circuit 816 are activated. Programming control circuit 816 dynamically updates values of the plurality of stimulation parameters in response to changes in the set of one or more stimulation waveforms, and transmits the plurality of stimulation parameters with the updated values to implantable stimulator 704.

FIG. 9 illustrates an embodiment of a system 960 for evoking and recording neural responses. System 960 can deliver neurostimulation to a target in a patient using a plurality of electrodes and can include a stimulation output circuit 912, a sensing input circuit (also known as a sensing circuit) 942, and a control circuit 914. Stimulation output circuit 912 can deliver the neurostimulation to evoke responses from the patient using an evoking configuration. The evoking configuration defines a stimulation electrode set including electrodes selected from the plurality of electrodes. In various embodiments, the evoking configuration can define various aspects of each stimulation electrode of the stimulation electrode set including, for example, any one or any combination of polarity (e.g., anode or cathode), pulse details (e.g. polarity order, relative size and shape of phases, interphases and duration, and active or passive recharge phases and order and duration of the recharge phases), and fractionalization (e.g., stimulation current specified in absolute value or as a percentage of a total stimulation current). Sensing input circuit 942 can sense one or more signals including the evoked responses using a recording configuration. The recording configuration defines a sensing electrode set including electrodes selected from the plurality of electrodes. In various embodiments, the recording configuration can define various aspects of each sensing electrode of the sensing electrode set including, for example, any one or any combination of physical arrangement (e.g., location relative to or distance from the stimulation electrode set), ganging (e.g. electrical connection of multiple physical electrodes), polarity (e.g., connected to inverting or noninverting input of a sensing input circuit), biasing or referencing voltage applied, gain and filter settings, and offset-compensation applied. Control circuit 914 can include a stimulation controller 962, a sensing controller 964, and a parameter adjuster 966. Stimulation controller 962 can control the delivery of the neurostimulation using stimulation parameters including the evoking configuration. Sensing controller 964 can control the sensing of the one or more signals including the evoked responses using sensing parameters including the recording configuration. Parameter adjuster 966 can determine an evoking-recording parameter set by evaluating a sequence of test evoking-recording parameter sets. The evoking-recording parameter set includes a set of the stimulation parameters suitable for controlling stimulation output circuit 912 to deliver the neurostimulation to evoke a target response and a set of the sensing parameters suitable for controlling sensing input circuit 942 to record the evoked target response. In various embodiments, the neural responses to be evoked and recorded using system 960 can include evoked potentials (EP), with evoked residential neural activities (ERNA) being a specific example when system 960 is implemented in a neurostimulation system for delivering DBS.

System 960 can be implemented in a neurostimulation system such as system 100 or 600. In various embodiments, system 960 is implemented in an implantable medical device, such as IPG 404, IPG 504, IPG 604, or implantable stimulator 704 as discussed in this document. For example, when system 960 is implemented in implantable stimulator 704, stimulation output circuit 212 can be configured to include stimulation circuit 912, sensing input circuit 742 can be configured to include sensing input circuit 942, and implant control circuit 714 can be configured to include control circuit 914. The plurality of electrodes using for delivering the neurostimulation can include electrodes 706 and 707. In various other embodiments, system 960 is implemented in an implantable medical device, such as IPG 404, IPG 504, IPG 604, or implantable stimulator 704 and an external programming device such as CP 630, RC 632, or external programming device 802 as discussed in this document. For example, when system 960 is implemented in implantable stimulator 704 and external programming device 802, stimulation output circuit 212 can be configured to include stimulation circuit 912, sensing input circuit 742 can be configured to include sensing input circuit 942, and implant control circuit 714 and interface control circuit 854 can be configured to include control circuit 914. In other words, control circuit 914 can be distributed in implant control circuit 714 and interface control circuit 854. The plurality of electrodes using for delivering the neurostimulation can include electrodes 706 and 707.

In various embodiments, the neurostimulation is delivered in the form of electrical stimulation pulses (referred to as neurostimulation pulses in this document), and system 960 is configured for delivering the neurostimulation pulses and sensing one or more signals including neural responses to the delivery of the neurostimulation pulses using the plurality of electrodes.

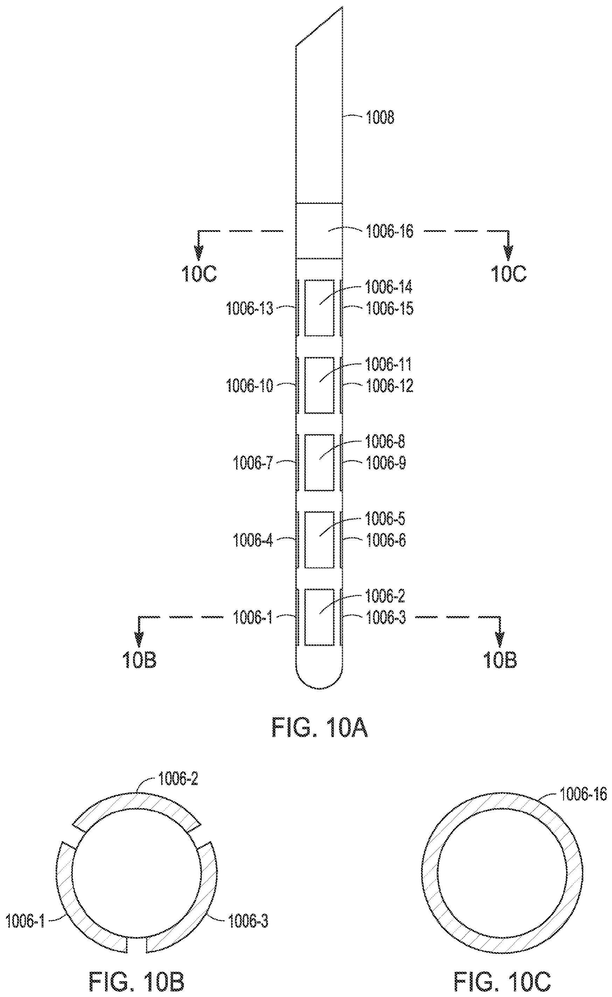

FIG. 10A-C illustrate an embodiment of a lead 1008 for use with a system for evoking and recording neural responses, such as system 960. Lead 1008 can represent an example of lead (or lead system) 208, 408A, 408B, 508, 608A, 608B, or 708. FIG. 10A is a side view of a distal portion of lead 1008. Illustrated in FIG. 10A as an example, lead 1008 includes an array of electrodes including electrodes (also referred to as contacts) 1006-1, 1006-2, ..., 1006-16. In this illustrated example, electrodes 1006-1, 1006-2, and 1006-3 can be collectively referred to as a first 3-segment electrode (which includes 3 electrodes, or contacts, radially distributed along a ring shape). FIG. 10B is cross-sectional view taken along line B-B showing the 3-segment electrode (without showing other details of lead 1008). Likewise, electrodes 1006-4, 1006-5, and 1006-6 form a second 3-segmental electrode; electrodes 1006-7, 1006-8, and 1006-9 form a third 3-segmental electrode; electrodes 1006-10, 1006-11, and 1006-12 form a fourth 3-segmental electrode; and electrodes 1006-13, 1006-14, and 1006-15 form a fifth 3-segmental electrode. Electrode 1006-16 can be referred to as a ring electrode (which includes a single contact having a ring shape). FIG. 10C is a cross-sectional view taken along line C-C showing the ring electrode (without showing other details of lead 1008). The electrode array with a ring electrode and 5 3-segment electrodes is shown in FIGS. 10A-C as an example. In various embodiments, the electrode array can have any structural and electrical configuration suitable for delivering neurostimulation (e.g., for effecting a desirable stimulation field). For example, various embodiments of lead 1008 can include one or more ring electrodes and one or more multi-segment electrodes. The one or more ring electrodes each include a single contact having a ring shape. The one or more multi-segment electrodes each include multiple contacts radially distributed along a ring shape.

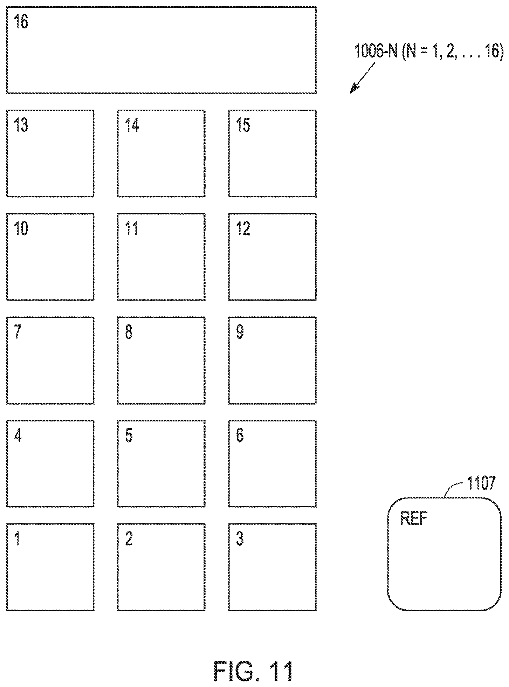

FIG. 11 illustrates an embodiment of the plurality of electrodes that can be used with system 960 for delivering neurostimulation to the target. Illustrated in FIG. 11 as an example, the plurality of electrodes include electrodes 1006-1, 1006-2, ..., 1006-16 (i.e., 1006-N, where N = 1, 2, ..., 16) on lead 1008 (shown in FIG. 11 as a flattened view) and a reference electrode 1107. Examples of reference electrode 1107 include a skin patch electrode (e.g., for use during an implantation procedure), a physical electrical clip connected to the metal portion of the canula above the head and within the stereotactic frame, such that canula is in contact with the inner tissue, an physically clipped connection to any other metal component in contact with appropriate inner tissue, and an electrode incorporated onto an implantable neurostimulator that is to be coupled to lead 1008 (e.g., electrodes 426, 428, and 707 as discussed in this document). In some embodiments, one or more of electrodes 1006-N can be used as reference electrode 1107 (e.g., for bipolar stimulation and/or differential sensing). In various embodiments, reference electrode 1107 can be placed in any location in or on the patient to provide the desirable referencing and/or biasing functions In this document, each electrode on a lead can be referred to as a "lead electrode" (e.g., each of lead electrodes 1006-N).

Referring back to FIG. 9, in various embodiments, stimulation output circuit 912 can include multiple stimulation channels, and sensing input circuit 942 multiple sensing channels. Stimulation controller 962 can control the delivery of the neurostimulation using the stimulation parameters for each stimulation channel of the multiple stimulation channels, thereby allowing for simultaneous delivery of the neurostimulation to multiple sites. Sensing controller 964 can control the sensing of multiple signals using the sensing parameters for each sensing channel of the multiple sensing channels, thereby allowing for simultaneous sensing of the evoked responses from multiple sites. This allows parameter adjuster 966 to evaluate multiple test evoking-recording parameter sets simultaneously.

As discussed above, the evoking-recording parameter set to be determined by parameter adjuster 966 includes a set of the stimulation parameters, which includes the evoking configuration, and a set of the sensing parameters, which includes the recording configuration. In various embodiments, the evoking configuration defines a stimulation electrode set including electrodes selected from the plurality of electrodes. In various embodiments, the evoking configuration further defines a fractionalization specifying a distribution of a current of the neurostimulation over the stimulation electrode set. The fractionalization can assign fraction of an amplitude of the neurostimulation to each electrode in the stimulation electrode set. In various embodiments, the fractionalization can be used to create a single volume of activation or multiple volumes of activation by the neurostimulation. The single volume of activation can be used to evoke neural responses from a single neural target (e.g., a tissue volume, fiber tract, or structure) or area of overlapping targets. The multiple volumes of activation is used to evoke one or more types of neural responses from multiple neural targets. In various embodiments, stimulation controller 962 provides for multiple independent current control (MICC), which controls the current of the neurostimulation on each electrode of the plurality of electrodes. In various embodiments, the plurality of electrodes includes lead electrodes and at least one reference electrode (e.g., lead electrodes 1006-N and reference electrode 1107 as illustrated in FIG. 11). The stimulation electrode set can include a cathode including one or more lead electrodes of the lead electrodes and an anode including (1) the reference electrode (known as monopolar stimulation), (2) one or more additional lead electrodes of the lead electrodes (known as bipolar stimulation), or (3) a reference electrode and one or more additional lead electrodes of the lead electrodes (known as anodic stimulation). In various embodiments, the set of the stimulation parameters further include, for example, stimulation channels and stimulation waveform parameters. When the neurostimulation is delivered in the form of electrical pulses, the stimulation waveform parameters can include, for example, pulse amplitude, pulse width, pulse frequency (also referred to as pulse rate), and pulse shape. In various embodiments, the recording configuration defines a sensing electrode set including electrodes selected from the plurality of electrodes. In various embodiments, the set of sensing parameters further include, for example, sensing channels and settings of sensing input circuit 942 for processing each of the sensed one or more signals. The processing of each sensed signal can include, for example, one or more of amplification of the sensed signal, filtering of the sensed signal, digitization of the sensed signal, averaging of the sensed signal, and extracting one or more features from the sensed signal. In various embodiments, the set of sensing parameters can define the recording configuration and one or more of the following, for example:
- one or more sensing channels to activate;
- sensing electrodes for each sensing channel;
- sampling frequency for each sensed signal (depending on the type of the signal);
- filter type and cutoff frequencies for each sensed signal (depending on the type of the signal);
- sensing time window for each sensed signal (duration of sensing, depending on the signal and the signal features of interest, e.g., response following a stimulus);
- whether each sensed signal is averaged (e.g., responses averaged for multiple stimuli);
- whether each sensed signal in its time sensing time window is weighted by a decaying factor varying with time prior to being averaged;
- whether each sensed signal or one or more signal features extracted from that signal is/are stored;
- type(s) of signal feature to be extracted from each sensed signal (e.g., range, curve length (CL), area under curve (AUC), frequency domain features); cross-channel features (coherence, cross-correlation, mutual information), or other statistical features;
- stimulation delivered for purpose of sensing with specific stimulation parameters; and
- default settings for different signals.

In various embodiments, parameter adjuster 966 can determine the evoking-recording parameter set for one or more algorithms related to the initial setting and/or adjustment of neurostimulation for a patient. Examples of such one or more algorithms include a lead checking algorithm for verifying lead (including electrodes) placement and/or functioning, a sweet spot searching algorithm for locating stimulation site(s), a sensing check algorithm for verifying functioning of the neurostimulation system for sensing evoked potentials, a closed-loop control algorithm for using sensed signal(s) to control the delivery of the neurostimulation, and/or a medication effect algorithm for monitoring effects of medications on evoked potentials. In one embodiment, parameter adjuster 966 determines the evoking-recording parameter set for one or more algorithms associated with a DBS therapy. In various embodiments, parameter adjuster 966 can determine the evoking-recording parameter set for any type of neurostimulation therapy where reliable sensing of evoked potentials is required.

In various embodiments, the evoking-recording parameter set to be determined using parameter adjuster 966 can include an evoking-recording configuration and a stimulation waveform parameter set. The evoking-recording configuration including a pair of the evoking configuration and the recording configuration. The stimulation waveform parameter sets including one or more adjustable stimulation waveform parameters to be used with the evoking-recording configuration. The sequence of test evoking-recording parameter sets to be evaluated using parameter adjuster 966 includes a sequence of test evoking-recording configurations and a sequence of test stimulation waveform parameter sets for each test evoking-recording configuration of the sequence of test evoking-recording configurations.

The evaluation of the sequence of test evoking-recording parameter sets can include a sequence of measurements each including evoking and recording of a neural response using a test evoking-recording parameter set. Parameter adjuster 966 can control the timing for each test evoking-recording parameter set to be evaluated. To evaluate each test evoking-recording parameter set, stimulation controller 962 can control stimulation output circuit 912 to deliver a burst of neurostimulation pulses, and sensing controller 964 can control sensing input circuit 942 to sense the one or more signals indicative of the response to the delivery of the burst of neurostimulation pulses. In various embodiments, parameter adjuster 966 can select the evoking-recording parameter set suitable for sensing evoked target responses from the sequence of test evoking-recording parameter sets based on a result of the evaluation. In various embodiments, parameter adjuster 966 can select a reliable evoking-recording parameter set for the evoked target responses using one or more sensing reliability criteria. Examples of such one or more sensing reliability criteria include an amplitude of the sensed evoked target response exceeding a threshold amplitude and a signal-to-noise ratio (SNR) of the sensed evoked target response exceeding a threshold SNR. In various other embodiments, the evoking-recording parameter set can be optimized for sensing the evoked target response, such as for a minimum stimulation energy to evoke the target response having an amplitude or SNR exceeding a threshold amplitude or SNR or for a maximum amplitude or SNR of evoked target response obtained using a specified stimulation energy. Parameter adjuster 966 can select an optimal evoking-recording parameter set for sensing the evoked target response from the sequence of test evoking-recording parameter sets.

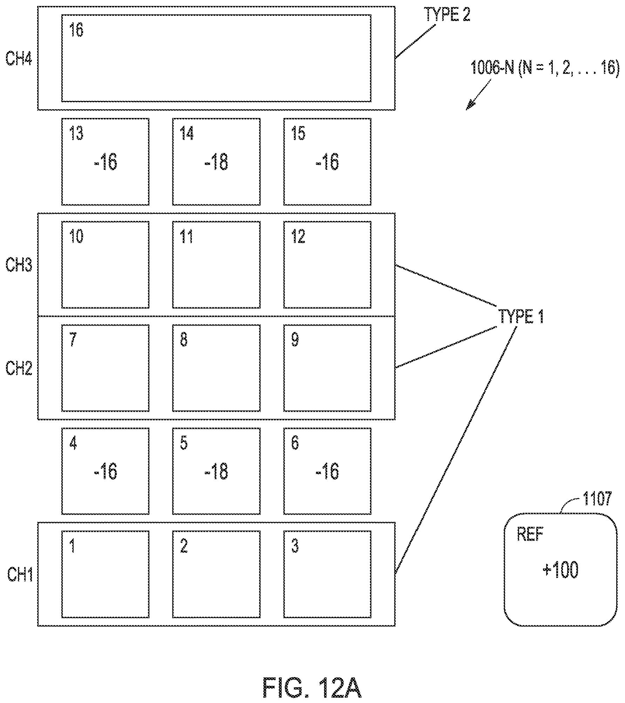

The sequence of test evoking-recording parameter sets can include one or more test evoking-recording parameter sets each specifying a single electrode set for the stimulation electrode set and the sensing electrode set and one or more test evoking-recording parameter sets each specifying different electrode sets for the stimulation electrode set and the sensing electrode set. In various embodiments, the sensing electrode set in each test evoking-recording parameter set can be specified by an electrode type and a location relative to the stimulation electrode set. For example, when using the plurality of electrodes of FIG. 11 (i.e., lead electrodes 1006-N and reference electrode 1107), the electrode type can be specified as either a ring electrode (1006-16) or a 3-segment electrode (all 3 contacts selected: 1006-1, 1006-2, and 1006-3; 1006-4, 1006-5, and 1006-6; 1006-7, 1006-8, and 1006-9; 1006-10, 1006-11, and 1006-12; or 1006-13, 1006-14, and 1006-15), and the location can be specified by the distance between the sensing electrode set and the stimulation electrode set. For example, such a distance can be coded as:
- 0: the same one or more electrodes used as the sensing electrode set and the stimulation electrode set;
- 1: the sensing electrode set directly adjacent to the stimulation electrode set; and
- 2: the sensing electrode set separated from the stimulation electrode set by one electrode.
This example for specifying the sensing electrode set is provided for illustrative rather than restrictive purposes. Any electrode in the plurality of electrodes used with system 960 can be selected to be included in the stimulation electrode set and/or the sensing electrode set of each evoking-recording parameter set. In various embodiments, the stimulation electrode set and the sensing electrode set can be specified in any way suitable for identifying the selected electrodes. In various embodiments, each contact in a multi-segment electrode can be specified individually. FIGS. 12A-F illustrate examples of evoking-recording configurations specifying different stimulation and sensing electrode sets selected from the plurality of electrodes of FIG. 11 (i.e., lead electrodes 1006-N and reference electrode 1107). In FIGS. 12A-F, the number with polarity (+/-) sign within each contact shows the percentage of the stimulation current flowing through that contact and whether the contact is used as the cathode (-) or the anode (+). In various embodiments, the location for the sensing electrode set can further indicate whether the sensing electrode set is between the anode(s) and the cathode(s) of the stimulation electrode set for bipolar stimulation or outside the bipolar stimulation electrical field or nearer to anode(s) or cathode(s).

With respect to the stimulation electrode set, the sensing electrode set, and the fractionalization, it is noted that in some embodiments, the stimulation electrodes can be chosen so as to affect a single neural structure, population, region, target, fiber tract, or network. When referring to Volume of Tissue Activated (VTA), in some embodiments, the stimulation electrodes can be chosen to create a single continuous or single effective VTA. In this manner, the complex set of active stimulating electrodes can intend to elicit a single effective response, to be captured by the sensing electrodes. In other embodiments, and especially when performing multi-channel simultaneous stimulation and sensing, more than one area of neural tissue is intended to be modulated, especially simultaneously or in short succession. In this manner, multiple discontinuous or separate VTAs can be used. These can then be sensed simultaneously by one or more sensing electrode sets, collecting more than one set of information about the response to stimulation at the same time. In this manner, in some embodiments, a single response is recorded by one or more sensing channels, each channel including one or more electrodes, and in other embodiments, multiple responses are recorded by one or more sensing channels, either simultaneously or in short succession.

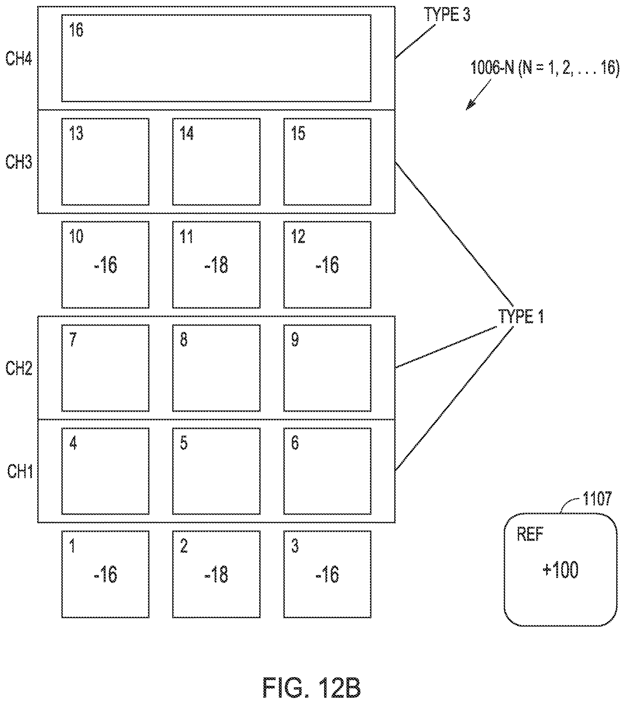

FIG. 12A illustrates a first example with a monopolar stimulation configuration. FIG. 12B illustrates a second example with a monopolar stimulation configuration. In the illustrated examples, four sensing channels are used to allow four test evoking-recording parameter sets specifying one stimulation electrode set and four sensing electrodes sets to be evaluated simultaneously. Three types of sensing electrode sets are evaluated in the examples illustrated in FIGS. 12A and 12B:
- Type 1: electrode type: 3-segment, location: 1;
- Type 2: electrode type: ring, location: 1; and
- Type 3: electrode type: ring, location: 2.

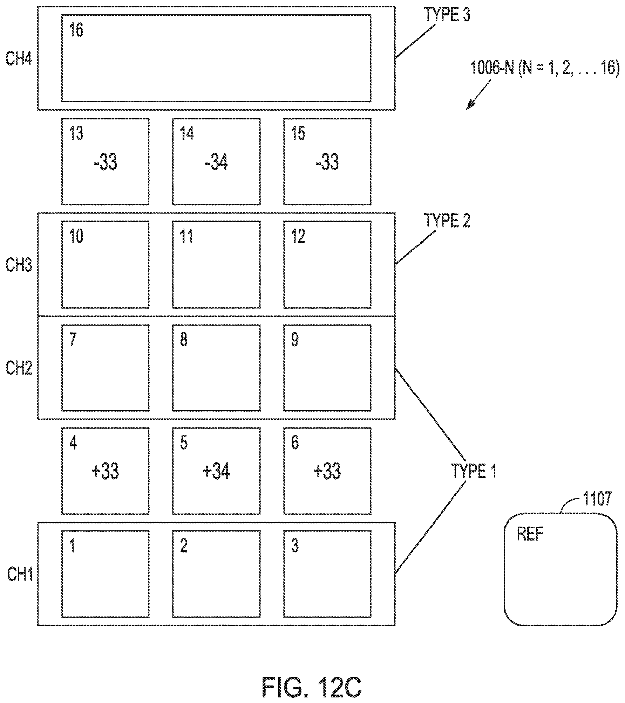

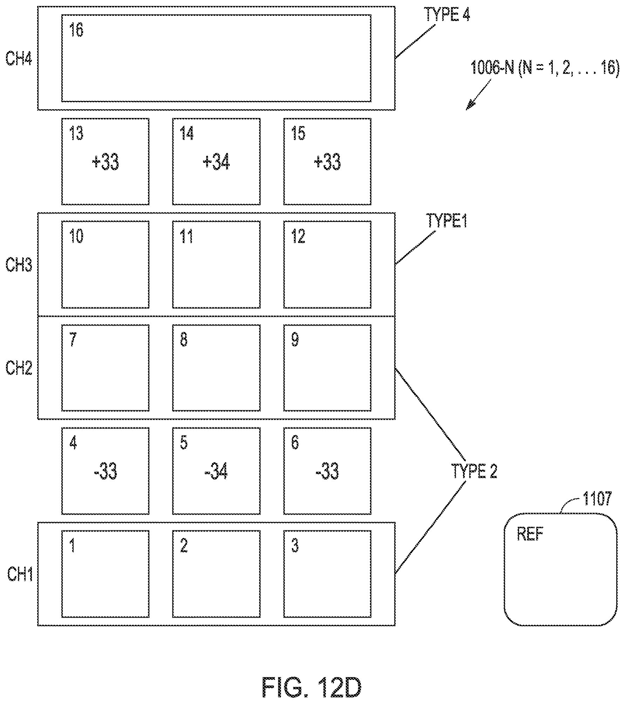

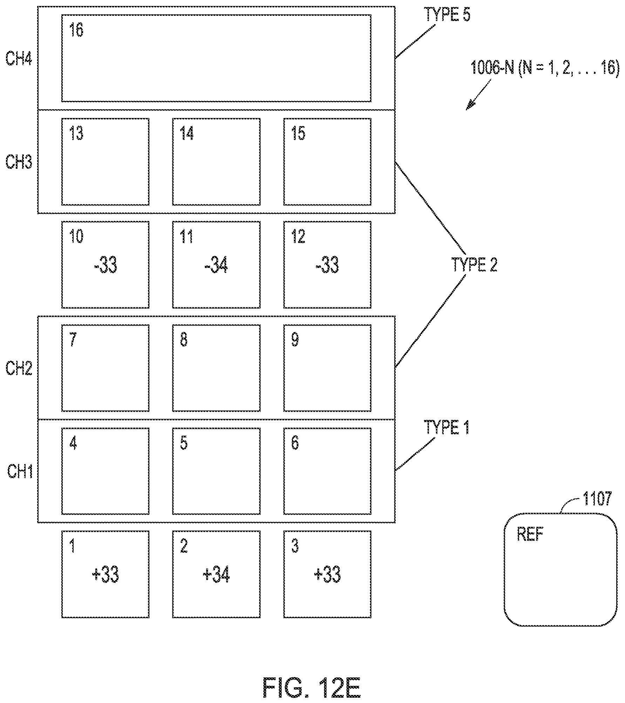

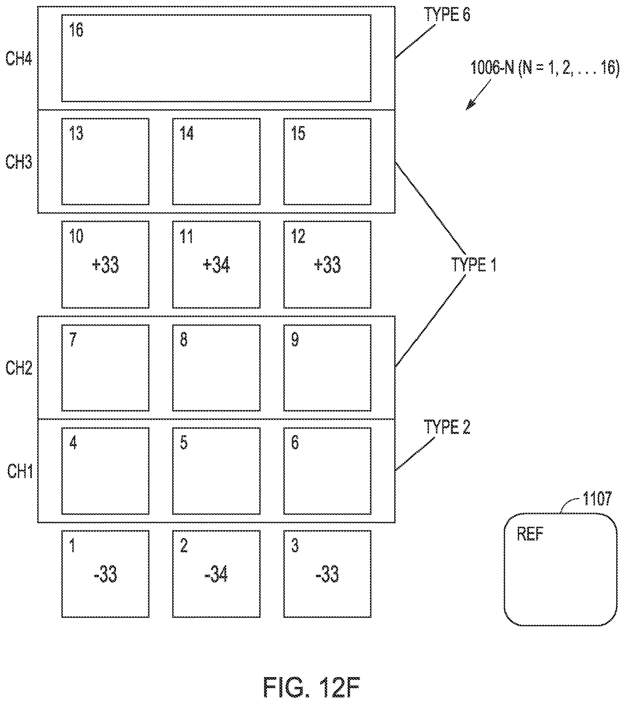

FIG. 12C illustrates a first example with a bipolar stimulation configuration. FIG. 12D illustrates a second example with a bipolar stimulation configuration. FIG. 12E illustrates a third example with a bipolar stimulation configuration. FIG. 12F illustrates a fourth example with a bipolar stimulation configuration. In the illustrated examples, four sensing channels are used to allow four test evoking-recording parameter sets specifying one stimulation electrode set and four sensing electrodes sets to be evaluated simultaneously. Six types of sensing electrode sets are evaluated in the examples illustrated in FIGS. 12C-12F:
- Type 1: electrode type: 3-segment, location: 1 from anode;
- Type 2: electrode type: 3-segment, location: 1 from cathode;
- Type 3: electrode type: ring, location: 1 from cathode;
- Type 4: electrode type: ring, location: 1 from anode;
- Type 5: electrode type: ring, location: 2 from cathode; and
- Type 6. electrode type: ring, location: 2 from anode.

As shown in FIGS. 12A-F, the sensing electrodes sets in the test evoking-recording parameter sets can be specified by the type that is defined by the electrode type and location. When the sensing electrodes sets are so specified, each type can include one or more sensing electrode sets (and hence correspond to one or more test evoking-recording parameter sets). For example, in FIGS. 12C, 12D, 12E, and 12F, two, one, one, and two sensing electrode sets fit into the description of type 1 sensing electrode set, respectively. Thus, there are six type 1 sensing electrode sets, which correspond to six test evoking-recording parameter sets.

In various embodiments, including these illustrated in FIGS. 12A-F, the test evoking-recording parameter sets can each define a type and a location for the reference electrode 1107. The type depends on the reference electrode used, such as a skin patch electrode or other type of patient-interfacing electrode, a clip, an alternate lead, or the electrode incorporated onto the implantable stimulator, as discussed above with reference to FIG. 11. Some types of the reference electrode can allow for temporary placement in or on the patient (e.g., the skin patch electrode) at a location particularly suitable for evaluating the test evoking-recording parameter sets.

Using the evoking-recording configurations illustrated in FIGS. 12A-F, equivalent stimulation current can be applied in all angular directions (e.g., one third in each direction). This allows for determination of the anatomical sensing location that provides the targeted neural response. Once the row or rows with sensing contacts exhibiting the targeted neural response are obtained, further refinements in this anatomical location can be achieved, for example by introducing variations in the percentage (fractionalization) of the current amplitude applied to each row of contacts, which allows an angular search of the targeted neural response. In this case, there will be more types electrode type definitions, as the different percentage of stimulation current is another variable introducing the additional types.

While one lead is used as an example in FIGS. 11 and 12A-F, the plurality of electrodes used in various embodiments (e.g., connected to system 960) can include lead electrodes selected from one lead or multiple leads. When compared to using a single lead, using multiple leads can provide more evoking-recording configurations that are substantially different in functional performances in evoking and recording targeted neural references. In addition to the larger variety of electrode combinations, multiple leads can provide more substantially different referencing and biasing options.

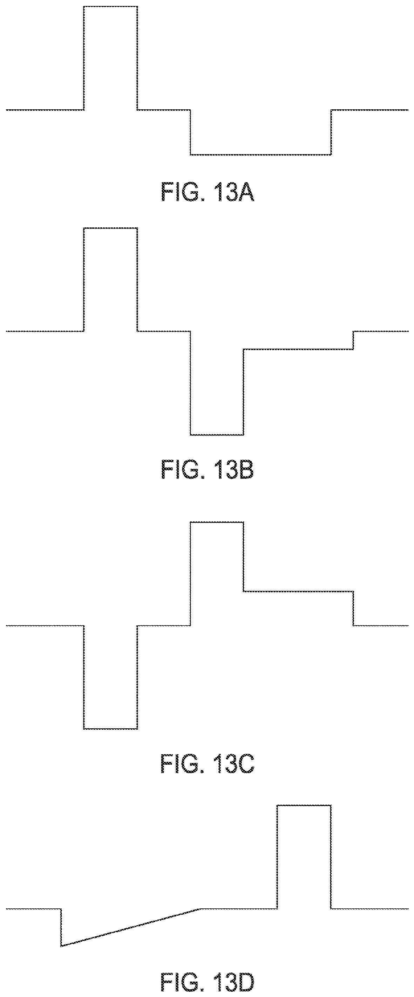

FIG. 13A-D illustrate examples of stimulation waveforms for use in a system for evoking and recording neural responses, such as system 960. Mismatch between electrode surface area or electrode-tissue interface can create DC offsets that induce amplifier saturation in sensing input circuit 942. FIG. 13A shows an example of a biphasic stimulation pulse waveform that can cause saturation in the sensed signal. An additional phase can be added to each pulse to negate such saturation. FIGS. 13B and 13C each show an example of stimulation pulse waveform that includes such an additional phase. Stimulation artifact in the sensed signal can be reduced to allow sensing input circuit 942 to be ready for sensing more quickly following delivery of each stimulation pulse. FIG. 13D shown an example of a waveform with a recharging phase for charge balance with minimal effect on the sensed signal. In various embodiments, waveform of the neurostimulation can be designed to minimize the stimulation artifact and/or other direct effect (rather than a response from the patient) in the sensed signal, allowing more time for sensing the neural response including the evoked potentials.

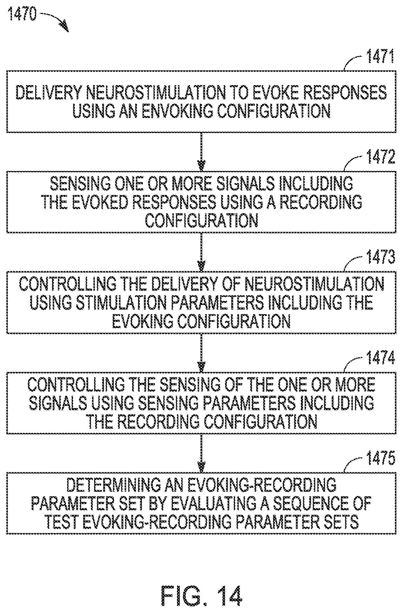

FIG. 14 illustrates an embodiment of a method 1470 for evoking and recording neural responses. Method 1470 can be performed using system 960 when system 960 is implemented in a neurostimulation system such as system 100 or 600, including their various embodiments as discussed in this document. In various embodiments, method 1470 can be performed for delivering neurostimulation to a patient through a plurality of electrodes, such as the plurality of electrodes illustrated in FIG. 11. Method 1470 can be performed to determining the evoking-recording parameter set in various neurostimulation therapies, including DBS therapies. In various embodiments, the evoking-recording parameter set is suitable or optimized for evoking and sensing evoked potentials such as ERNA.

Steps 1471-1475 of method 1470 can be performed in any temporal order that is logical and meaningful based on practical and design considerations. For example, steps 1473 and 1474 can be performed simultaneously, and each step can be repeatedly performed as needed until one or more satisfactory evoking-recording parameter sets are obtained.

At 1471, the neurostimulation is delivered to evoke responses from the patient using a stimulation output circuit and an evoking configuration. The evoking configuration defines a stimulation electrode set including electrodes selected from the plurality of electrodes. In various embodiments, the neurostimulation is delivered simultaneously through multiple stimulation channels of the stimulation output circuit using multiple evoking configurations each defining a different stimulation electrode set for use with one of the multiple stimulation channels. In various embodiments, in additional to the stimulation electrode set, the evoking configuration defines a fractionalization specifying a distribution of a current of the neurostimulation over the stimulation electrode set.

At 1472, one or more signals including the evoked responses are sensed using a sensing input circuit and a recording configuration. The recording configuration defines a sensing electrode set including electrodes selected from the plurality of electrodes. In various embodiments, multiple signals are sensed simultaneously through multiple sensing channels of the sensing input circuit using multiple sensing configurations each defining a different sensing electrode set for use with one of the multiple sensing channels. Multiple test evoking-recording parameter sets can be evaluated simultaneously using the multiple sensing channels of the sensing input circuit. In some embodiments, multiple test evoking-recording parameter sets can be evaluated simultaneously using the multiple sensing channels of the sensing input circuit and the multiple stimulation channels of the stimulation output circuit.

At 1473, the delivery of the neurostimulation is controlled using stimulation parameters including the evoking configuration. In various embodiments, the evoking configuration can include the stimulation electrode set selected from a reference electrode (e.g., reference electrode 1107) of the plurality of electrodes and lead electrodes (e.g., lead electrodes 1006-N) of the plurality of electrodes. In various embodiment, an electrical current of the neurostimulation can be controlled individually for each of the reference electrode and the lead electrodes (e.g., reference electrode 1107 or each of the 16 lead electrodes 1006-N). In various embodiments, selecting the stimulation electrode set for each evoking configuration can include, for example, selecting one or more lead electrodes of the lead electrodes to function as a cathode and
- selecting the reference electrode to function as an anode;
- selecting one or more additional lead electrodes of the lead electrodes to function as an anode; or
- selecting the reference electrode and one or more additional lead electrodes of the lead electrodes to function as an anode.

At 1474, the sensing of the one or more signals including the evoked responses is controlled using sensing parameters including the recording configuration. In various embodiments, the evoking configuration can include the sensing electrode set selected from the same plurality of electrodes from which the stimulation electrode set is selected from, i,e., the reference electrode (e.g., reference electrode 1107) and the lead electrodes (e.g., lead electrodes 1006-N). In various embodiments, the sensing electrode set can be specified by an electrode type (e.g., the ring electrode or the multi-segment electrode) and an electrode location relative to the stimulation electrode set used to evoke the response to be recorded using the sensing electrode set.

At 1475, an evoking-recording parameter set is determined by evaluating a sequence of test evoking-recording parameter sets. The evoking-recording parameter set includes a set of the stimulation parameters (including the evoking configuration) suitable for controlling the delivery of the neurostimulation to evoke a target response and a set of the sensing parameters (including the recording configuration) suitable for controlling the sensing of the one or more signals to record the evoked target response. In one embodiment, the evoking-recording parameter set is optimized for evoking and recording a specified target response. The evoking-recording parameter set can be optimized, for example:
- for a minimum intensity of the neurostimulation that results in a parameter (e.g., amplitude or SNR) of the recorded evoked target response reaching or exceeding a specified threshold, or
- for a maximum parameter (e.g., amplitude or SNR) of the recorded evoked target response resulting from a specified (fixed) intensity of the neurostimulation.

Each test evoking-recording parameter set can include an evoking-recording configuration, which includes a pair of the evoking configuration and the recording configuration and a stimulation waveform parameter set. The evaluation of the sequence of test evoking-recording parameter sets can include evaluating a sequence of test evoking-recording configurations and a sequence of test stimulation waveform parameter sets for each test evoking-recording configuration of the sequence of test evoking-recording configurations. The sequence of test evoking-recording parameter sets can be evaluated by delivering a burst of pulses of the neurostimulation and sensing the one or more signals according to each test evoking-recording parameter set of the sequence of test evoking-recording parameter sets. The evoking-recording parameter set can be selected from the sequence of test evoking-recording parameter sets based on a result of the evaluation. In various embodiments, the sequence of test evoking-recording parameter sets include multiple evoking-recording configurations using different recording electrode sets selected from lead electrodes on each lead for recording the evoked target response.

Given the number of possible electrode combinations, testing all possible evoking-recording configurations as well as their combinations with possible stimulation waveform parameters with a patient, often in an operation room during device implantation and initial setting, is too time-consuming and hence impractical. The time required for evaluating the sequence of evoking-recording parameter sets also include, for example, the number of pulses required to evoke the target responses suitable for measurement and settlement of the sensing input circuit to allow for the measurement. In various embodiments, one or any combination of the following example methods can be applied for evaluating the sequence of test evoking-recording parameter sets in an efficient and effective manner:
- using a minimum number of number of pulses in each burst for evoking the evoked target responses (e.g., by stopping the burst upon stable detection of the evoked target responses;
- selecting stimulation waveform shape(s) for reducing stimulus artifacts in the sensed signals (e.g., using additional phase(s) in each biphasic stimulation pulse and/or using the recharging phase of each biphasic stimulation pulse for charge balance with little or no effect on the sensed signal(s);
- testing multiple test evoking-recording parameter sets simultaneously using multiple sensing channels;
- testing multiple test evoking-recording parameter sets simultaneously using multiple sensing channels and multiple stimulation channels when different target responses can be evoked and recorded independently and simultaneously;

- limiting the number of the test evoking-recording parameter sets in the sequence by identifying and including representative types of the evoking-recording configurations to be tested;
- limiting the evoking-recording configurations to be tested to one or more regions of interest for using prior information (e.g., user input, surgical plan, data obtained by imaging, and/or results from one or more previous evaluations);
- reducing or minimizing the number of changes in the recording configuration in the sequence of test evoking-recording parameter sets (e.g., by evaluating all the test stimulation parameter sets in the sequence that share the same recording configuration before proceeding to test stimulation parameter set having a different recording configuration, and/or evaluating all the stimulation waveform parameters associated with the same evoking-recording configuration before proceeding to a different evoking-recording configuration); and
- skipping one or more test evoking-recording parameter sets of the sequence as results from evaluating similar test evoking-recording parameter sets become available and make the potential results from the one or more test evoking-recording parameter sets predictable (e.g., when the results diminish expectation from the one or more test evoking-recording parameter sets).

It is to be understood that the above detailed description is intended to be illustrative, and not restrictive. Other embodiments will be apparent to those of skill in the art upon reading and understanding the above description. The scope of the invention should, therefore, be determined with reference to the appended claims, along with the full scope of equivalents to which such claims are entitled.
Further preferrred embodiments are described by the following items:
1. A system for delivering neurostimulation to a patient using a plurality of electrodes, comprising:
   a stimulation output circuit configured to deliver the neurostimulation to evoke responses from the patient using an evoking configuration defining a stimulation electrode set including electrodes selected from the plurality of electrodes:
   a sensing input circuit configured to sense one or more signals including the evoked responses using a recording configuration defining a sensing electrode set including electrodes selected from the plurality of electrodes: and
   a control circuit including:
      a stimulation controller configured to control the delivery of the neurostimulation using stimulation parameters including the evoking configuration;
      a sensing controller configured to control the sensing of the evoked responses using sensing parameters including the recording configuration; and
      a parameter adjuster configured to determine an evoking-recording parameter set by evaluating a sequence of evoking-recording parameter sets, the evoking-recording parameter set including a set of the stimulation parameters suitable for controlling the stimulation output circuit to deliver the neurostimulation to evoke a target response and a set of the sensing parameters suitable for controlling the sensing input circuit to record the evoked target response.
2. The system according to item 1, wherein the parameter adjuster is configured to determine the evoking-recording parameter set for controlling the stimulation output circuit and the sensing input circuit in deep brain stimulation.
3. The system according to any of the preceding items, wherein the stimulation output circuit comprises multiple stimulation channels, and the evoking configuration defines a different stimulation electrode set for each channel of the multiple stimulation channels to allow for simultaneous delivery of the neurostimulation using different stimulation electrode sets.
4. The system according to any of the preceding items, wherein the sensing input circuit comprises multiple sensing channels, and the recording configuration defines a different sensing electrode set for each channel of the multiple sensing channels to allow for simultaneous sensing of multiple signals using different sensing electrode sets.
5. The system according to item 4, wherein the parameter adjuster is configured to evaluate multiple evoking-recording parameter sets of the sequence of evoking-recording parameter sets simultaneously using two or more sensing channels of the multiple sensing channels.
6. The system according to any of the preceding items, wherein the evoking configuration further defines a fractionalization specifying a distribution of a current of the neurostimulation over the stimulation electrode set.
7. The system according to any of the preceding items, wherein the stimulation electrode set comprises electrodes selected from the plurality of electrodes for monopolar stimulation.
8. The system according to any of the preceding items, wherein the stimulation electrode set comprises electrodes selected from the plurality of electrodes for bipolar stimulation.
9. The system according to any of the preceding items, wherein the stimulation electrode set comprises electrodes selected from the plurality of electrodes for anodic stimulation.
10. The system according to any of the preceding items, wherein the stimulation output circuit is configured to deliver pulses of the neurostimulation, and the stimulation controller is configured to control the delivery of the pulses using the stimulation parameters including the evoking configuration and waveform parameters defining waveform of the pulses.
11. The system according to any of the preceding items, wherein the parameter adjuster is configured to identify an optimal evoking-recording parameter set from the sequence of evoking-recording parameter sets.
12. The system according to any of the preceding items, wherein the parameter adjuster is configured to reduce a number of the evoking-recording parameter sets in the sequence of evoking-recording parameter sets using prior information.
13. The system according to item 12, wherein the parameter adjuster is configured to reduce a number of changes in the recording configuration in the sequence of evoking-recording parameter sets.
14. The system according to any of the preceding items, wherein the parameter adjuster is configured to evaluate each evoking-recording parameter set of the sequence of evoking-recording parameter sets by causing the stimulation output circuit to deliver a burst of neurostimulation pulses and the sensing input circuit to sense the one or more signals using the each evoking-recording parameter set and to minimize a number of pulses in the burst of neurostimulation pulses while allowing for reliable sensing of the evoked target response.
15. The system according to any of the preceding items, wherein each evoking-recording parameter set in the sequence of evoking-recording parameter sets includes a pair of the stimulation electrode set and the sensing electrode set, the sensing electrode set of the pair defined by an electrode type and a location relative to the stimulation electrode set of the pair.

## Claims

1. A system for delivering neurostimulation to a patient using a plurality of electrodes, comprising:
a stimulation output circuit configured to deliver the neurostimulation to evoke responses from the patient using an evoking configuration defining a stimulation electrode set including electrodes selected from the plurality of electrodes;
a sensing input circuit configured to sense one or more signals including the evoked responses using a recording configuration defining a sensing electrode set including electrodes selected from the plurality of electrodes; and
a control circuit configured to control the delivery of the neurostimulation using stimulation parameters defining the evoking configuration, to control the sensing of the evoked responses using sensing parameters defining the recording configuration, and to adjust the stimulation parameters and the sensing parameters according to a sequence of evoking-recording parameter sets each including a set of the stimulation parameters determined for controlling the stimulation output circuit to deliver the neurostimulation to evoke a target response and a set of the sensing parameters determined for controlling the sensing input circuit to record the evoked target response.

2. The system according to any of the preceding claims, wherein the control circuit is configured to determine a suitable evoking-recording parameter set for evoking and recording the target response by evaluating the sequence of evoking-recording parameter sets.

3. The system according to claim 2, wherein the control circuit is configured to determine an evoking-recording parameter set suitable for evoking and recording evoked resonant neural activity in deep brain stimulation.

4. The system according to claim 2, wherein the control circuit is configured to select an optimal evoking-recording parameter set for sensing the evoked target response from the sequence of evoking-recording parameter sets.

5. The system according to any of claims 2 to 4, wherein the control circuit is configured to evaluate multiple evoking-recording parameter sets of the sequence of evoking-recording parameter sets simultaneously using two or more sensing channels of the multiple sensing channels.

6. The system according to any of the preceding claims, further comprising the plurality of electrodes and a lead configured to be coupled to the stimulation output circuit, wherein the plurality of electrodes includes a reference electrode and lead electrodes, the lead electrodes each include one or more contacts and incorporated onto the lead, and the control circuit is configured to control an electrical current of the neurostimulation individually for the reference electrode and each contact of the contacts of the lead electrodes.

7. The system according to claim 6, wherein the sequence of evoking-recording parameter sets comprises a set of the stimulation parameters defines an evoking configuration including one or more lead electrodes of the lead electrodes to function as a cathode for delivering the neurostimulation.

8. The system according to claim 7, wherein the evoking configuration further comprises the reference electrode to function as an anode for delivering the neurostimulation.

9. The system according to claim 7, wherein the evoking configuration further comprises one or more additional lead electrodes of the lead electrodes to function as an anode for delivering the neurostimulation.

10. The system according to claim 7, wherein the evoking configuration further comprises the reference electrode and one or more additional lead electrodes of the lead electrodes to function as an anode for delivering the neurostimulation.

11. The system according to any of the preceding claims, wherein the sequence of evoking-recording parameter sets specifies a temporal order of the evoking-recording parameter sets that is determined for reducing a duration of the delivery of the neurostimulation according to the sequence of evoking-recording parameter sets.

12. The system according to claim 11, wherein the control circuit is configured to reduce a number of the evoking-recording parameter sets in the sequence of evoking-recording parameter sets using prior information.

13. The system according to any of claims 11 and 12, wherein the sequence of evoking-recording parameter sets is determined by minimizing a number of changes in the recording configuration in the sequence of evoking-recording parameter sets.

14. The system according to claims 13, wherein the temporal order of the evoking-recording parameter sets allows for evaluation of all stimulation parameter sets for each evoking-recording configuration before switching to next evoking-recording configuration in the sequence of evoking-recording parameter sets.

15. The system according to claim 13, wherein the temporal order of the evoking-recording parameter sets allows for evaluation of all the evoking-recording parameter sets having a common recording configuration before evaluating evoking-recording parameter sets having a different recording configuration.
